(19) Europäisches Patentamt
European Patent Office
Office européen des brevets

(11) **EP 3 672 641 B1**

(12) **EUROPEAN PATENT SPECIFICATION**

(45) Date of publication and mention
of the grant of the patent:
**22.09.2021 Bulletin 2021/38**

(21) Application number: **18762605.6**

(22) Date of filing: **23.08.2018**

(51) Int Cl.:
*A61K 47/42* (2017.01)        *A61K 39/395* (2006.01)
*A61K 9/00* (2006.01)        *A61K 9/06* (2006.01)
*A61K 39/00* (2006.01)        *C07K 16/22* (2006.01)

(86) International application number:
**PCT/GB2018/052400**

(87) International publication number:
**WO 2019/038552 (28.02.2019 Gazette 2019/09)**

(54) **COMPOSITION COMPRISING VEGF ANTAGONISTS AND A CATIONIC PEPTIDE AND USES THEREOF**

ZUSAMMENSETZUNG MIT VEGF-ANTAGONISTEN UND EINEM KATIONISCHEN PEPTID SOWIE VERWENDUNGEN DAVON

COMPOSITION COMPRENANT DES ANTAGONISTES DU VEGF ET UN PEPTIDE CATIONIQUE ET SES UTILISATIONS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **25.08.2017 GB 201713724**

(43) Date of publication of application:
**01.07.2020 Bulletin 2020/27**

(73) Proprietor: **UCL BUSINESS LTD**
**London W1T 4TP (GB)**

(72) Inventors:
• **CORDEIRO, Francesca**
**London EC1V 9EL (GB)**
• **DAVIS, Benjamin Michael**
**London EC1V 9EL (GB)**
• **GRGIC, Ljuban**
**London EC1V 9EL (GB)**
• **SOMAVARAPU, Satyanarayana**
**London WC1N 1AX (GB)**

(74) Representative: **J A Kemp LLP**
**14 South Square**
**Gray's Inn**
**London WC1R 5JJ (GB)**

(56) References cited:
**US-A1- 2005 042 753**

• **ROSENFELD PHILIP J ET AL: "Ranibizumab for neovascular age-related macular degeneration", THE NEW ENGLAND JOURNAL OF MEDICINE, - NEJM -, MASSACHUSETTS MEDICAL SOCIETY, US, vol. 355, no. 14, 5 October 2006 (2006-10-05), pages 1419-1431, XP002506588, ISSN: 0028-4793, DOI: 10.1056/NEJMOA054481**
• **MIE KRISTENSEN ET AL: "Applications and Challenges for Use of Cell-Penetrating Peptides as Delivery Vectors for Peptide and Protein Cargos", INTERNATIONAL JOURNAL OF MOLECULAR SCIENCES, vol. 17, no. 185, 30 January 2016 (2016-01-30), page 185, XP055449924, DOI: 10.3390/ijms17020185**
• **IWASE YUKO ET AL: "Use of a non-covalent cell-penetrating peptide strategy to enhance the nasal delivery of interferon beta and its PEGylated form", INTERNATIONAL JOURNAL OF PHARMACEUTICS, ELSEVIER, NL, vol. 510, no. 1, 22 June 2016 (2016-06-22) , pages 304-310, XP029659372, ISSN: 0378-5173, DOI: 10.1016/J.IJPHARM.2016.06.054**
• **KAMEI N ET AL: "Usefulness of cell-penetrating peptides to improve intestinal insulin absorption", JOURNAL OF CONTROLLED RELEASE, ELSEVIER, AMSTERDAM, NL, vol. 132, no. 1, 24 November 2008 (2008-11-24), pages 21-25, XP025610273, ISSN: 0168-3659, DOI: 10.1016/J.JCONREL.2008.08.001 [retrieved on 2008-08-06]**

SEQUENCE LISTING

<110> UCL Business PLC

<120> FORMULATION

<130> N411074WO

<150> 1713724.1
<151> 2017-08-25

<160> 1

<170> PatentIn version 3.5

<210> 1
<211> 8
<212> PRT
<213> Artificial Sequence

<220>
<223> R8 peptide

<400> 1

```
Arg Arg Arg Arg Arg Arg Arg Arg
1               5
```

**Description**

**FIELD OF THE INVENTION**

The invention relates to a composition comprising Ranibizumab and the R8 peptide, and said composition for use in the treatment or prevention of vasculoproliferative conditions, particularly in the eye.

**BACKGROUND OF THE INVENTION**

Anti-Vascular Endothelial Growth Factors (anti-VEGFs) have revolutionised the management of age-related macular degeneration (AMD). Ranibizumab (Lucentis) intraocular injections are the most common National Institute for Health and Care Excellence (NICE)-approved anti-VEGF treatments, although successful injection regimens are very expensive (National Health Service (NHS) estimate £85 million/year). In addition to the increasing health-service burden, injections are associated with significant ocular complications including endophthalmitis, retinal detachment, cataract and uveitis, and are unappealing to patients. There is a wealth of clinical data supporting the efficacy and safety of Lucentis. Like the other monoclonal antibody treatments in this category it presently requires administration by a specialist clinician via intravitreal injection which is unpleasant for the patient and can be associated with infection, bleeding and retinal detachment.

Rosenfeld P et al., (2006) NEJM, 355, pages 1419-1431 describes Ranibizumab for neovascular age-related macular degeneration.

**SUMMARY OF THE INVENTION**

The present invention describes a novel formulation to dramatically enhance the amount of anti-VEGF reaching intraocular tissues via topical administration. It makes use of a small cationic peptide (R8, oligoarginine, RRRRRRRR (SEQ ID NO: 1)). Until now, R8 has been characterized as a cell-penetrating peptide (CPP). CPPs are defined as peptides which allow passage across cell membrane barriers allowing intracellular uptake of drugs (Ye et al. Int. J. Mol. Sci. 2016, 17(11), 1892). Our studies confirm that R8 promotes corneal uptake *in vivo* and *in vitro,* where passage of Lucentis was demonstrated through an intact cellular membrane barrier.

However, a surprising finding was that R8 promoted anti-VEGF delivery through corneal cells. This was demonstrated using a transcytosis assay and measuring the change in drug permeability coefficient ($P_{app}$) of Avastin and Lucentis in the presence of R8/ST-R8. $P_{app}$ of both anti-VEGFs was considerably increased in the presence of R8/ST-R8. This enhancement was found to occur in a concentration-dependent manner, with a molar ratio of between 40:1 and 200:1 R8 to antagonist being effective, and a molar ratio of between 50:1 to 80:1 R8 to antagonist being particularly effective.

Further investigation showed that passage across the corneal cells was non-toxic with R8 at all concentrations studied, and only toxic with ST(Stearyl)-R8 when the molar ratio >40:1. No changes were recorded in the transepithelial resistance (TEER), indicating involvement of a transcellular mechanism, as the corneal barrier integrity remained intact. This is again a new finding as previous studies have suggested that poly-arginine (36 kDa) polypeptides induced reversible disruption of tight junctions manifested as a change in TEER (Nemoto et al. Biol Pharm Bull. 2007 Sep;30(9):1768-72.). Additionally, these assays clearly showed that presence of R8 does not interfere with the VEGF binding activity. Finally, further functional assessment of anti-VEGF activity was performed *in vivo* in rabbits where only one eye was dosed with topical instillation of 10 mg/mL Lucentis compared to 10 mg/mL Lucentis/ 50 mg/mL R8. Measurements at 2 hours after topical application showed R8 to significantly increase levels of Lucentis in the vitreous and retina/choroid of freshly enucleated eyes (10 v 215 ng/g tissue).

The unique aspects of this approach is its simplicity as it does not require complex formulation or expensive constituents; simply mixing lyophilised peptide with VEGF antagonist such as Lucentis at the optimal molar ratio enables delivery of therapeutically relevant concentration to posterior segment tissues.

The invention uses Lucentis (Ranibizumab), and includes a novel formulation enabling administration as an eye-drop. This will:

- Avoid the unpleasant patient experience and potential adverse effects of intravitreal injection
- Enable self-administration at home
- Avoid costs associated with clinic visits for treatment and management of injection-related adverse effects
- Enable a potential dosing advantage (and potentially lower cost of therapy).
- Half-life of ranibizumab in the eye is reported to be 7.19 days. A high dose must therefore be used in order to maintain a therapeutic concentration at the back of the eye for the full month prior to the next injection.

The addition of small cationic peptides (like R8) to other drug proteins (antibodies, antibody fragments, therapeutic proteins) would similarly enhance the ability of these factors to be transported across biological barriers in a similar manner to that presented for Lucentis.

Our studies indicate that ~70% of the absorbed topically applied dose reaches intraocular tissues from topical rather than systemic absorption - a highly desirable pharmacodynamic property. No reports of ocular irritation or injury were recorded from rabbit *in vivo* studies and both agents were well tolerated using an *in vitro* model of the corneal epithelium (HCE-S cells).

Aggregation of the R8 peptide and Ranibizumab in the composition of the invention was reduced by the addition of Trehalose to the formulation. A concentration of 600 mg/ml Trehalose was found to be particularly effective.

Finally, in addition to high corneal permeation, enhancing ocular drug contact time is a recognised strategy for enhancing delivery of topically applied drugs to posterior ocular tissues. In the present invention enhancement of ocular drug contact time was achieved by formulating the aforementioned Ranibizumab and R8 peptide mixture with a viscosity enhancing agent such as a polymer or hydrogel. Incorporation of the Ranibizumab/R8 combination in a thermosetting hydrogel comprising 20% (w/v) Lutrol F127, dramatically enhanced the delivery of anti-VEGF to posterior ocular tissues compared to the previously described liquid eye drop formulation. The inventors propose that this effect will not be limited to Lutrol-F127 containing hydrogels and would also be observed for other bio-compatible viscosity enhancers, combinations thereof (e.g. hyaluronic acid, hypermellose, etc) or other biocompatible hydrogel gel-forming materials.

The inventors have also found that the addition of a mucoadhesive polymer, such as chitosan, to the formulation enhances the delivery of the formulation to posterior ocular tissues.

Thus, the invention provides:

A composition comprising the peptide RRRRRRRR (SEQ ID NO: 1) and Ranibizumab, wherein the molar ratio of peptide to Ranibizumab is at least 40:1.

The invention also provides:

The composition of the invention as defined herein, for use in the prevention or treatment of a condition characterised by VEGF dysfunction.

The invention also provides:

The composition of the invention as defined herein, for use in the prevention or treatment of a vasculoproliferative condition of the eye.

The invention also provides:

A kit comprising the composition of the invention as defined herein.


## BRIEF DESCRIPTION OF THE DRAWINGS

**Figure 1 - *In-vitro* measurement of transcellular passage of anti-VEGF** Transwell assay allows assessment of anti-VEGF transcytosis using TER as a measure of barrier integrity. TEER - Transepithelial electrical resistance describes the electrical resistance of the HCE-S corneal epithelial barrier. If the barrier remains intact, this value will not decline over the course of an experiment.

**[A]** Illustration of assay used. **[B]** Results demonstrating that the presence of R8 (Stearyl conjugated, ST-R8) enhanced the delivery of Lucentis across this corneal barrier model. (Two-way ANOVA with Bonferroni post-hoc tests **p < 0.01, ***p < 0.001).

**Figure 2 -Addition of R8 and Lucentis has no effect on corneal epithelial barrier integrity (no TEER decline)**

**Figure 3 - A ratio of R8 to Lucentis of 40:1 or more significantly enhances corneal transcytosis *in vitro***

A surprising finding in our *in vitro* studies was that there is evidence that R8 promoted delivery across corneal cells. Another method to monitor the extent of delivery across corneal barrier models is to calculate the drug permeability coefficient ($P_{app}$) as described in Davis et al 2014. R8 (=> 40:1 ratio) enhances Lucentis transcytosis (*p < 0.05, **p < 0.01). The effect of the ratio is surprising as previous work (de Cogan et al, 2017) has suggested a 25:1 molar ratio of R7 to Avastin is sufficient.

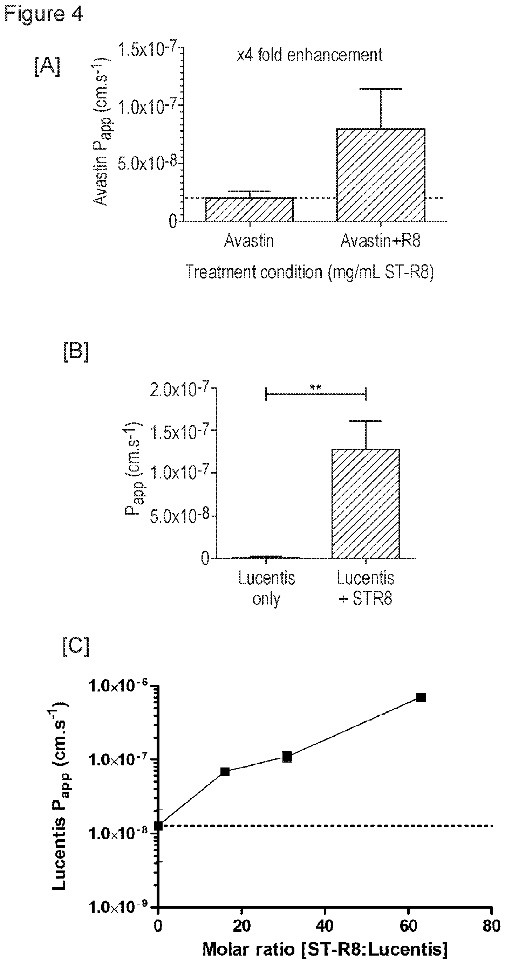

**Figure 4 - R8 enhances delivery of VEGF antagonists across corneal barriers *in vitro* in a concentration dependent manner.**

The presence of R8 (ST-R8) aided the transport of the anti-VEGFs **[A]** Avastin and **[B]** Lucentis across the HCE-S barrier (two tailed T-test, P = 0.0096). **[C]** Stearyl-R8 enhanced the delivery of the anti-VEGF Lucentis across the HCE-S barrier in a concentration dependent manner. One-way ANOVA with Bonferroni's Multiple Comparison Test P = 0.0307.

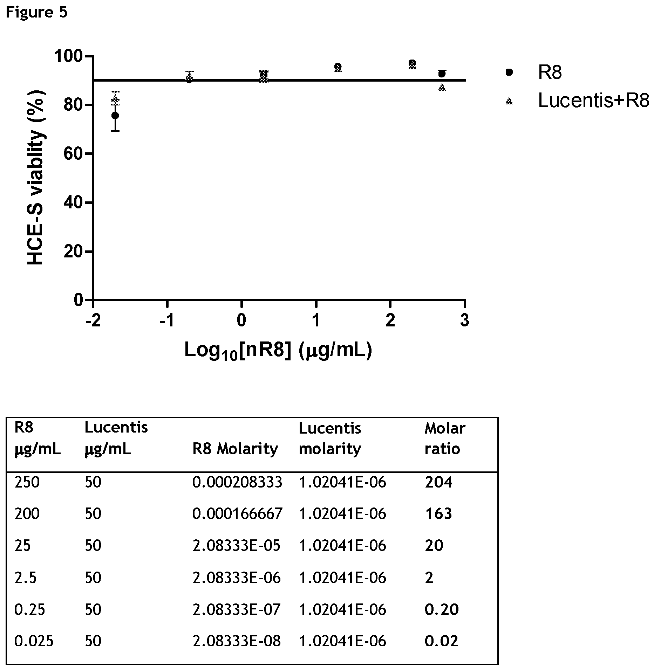

**Figure 5 - HCE-S Toxicity assay with R8/Lucentis**

R8 and Lucentis (maximal 200/1 ratio) combinations were well tolerated by HCE-S cells *in vitro* at all concentrations tested. Table shows molar ratios of R8 to Lucentis used at each point of the assay.

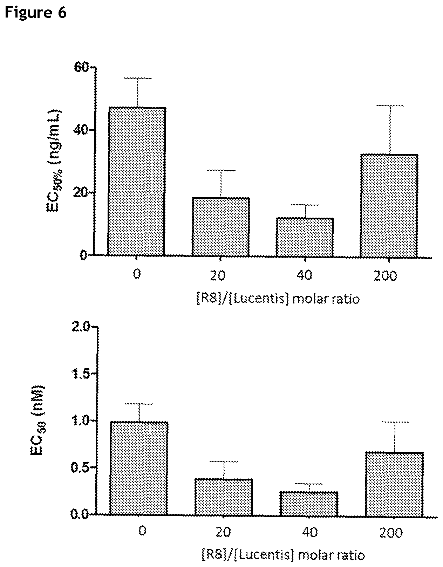

**Figure 6 - R8 does not adversely influence VEGF$_{165}$ binding** A functional ELISA was used to monitor the affinity of Lucentis in the presence of varying concentrations of R8.

Addition of R8 to Lucentis did not negatively impact VEGF binding activity.

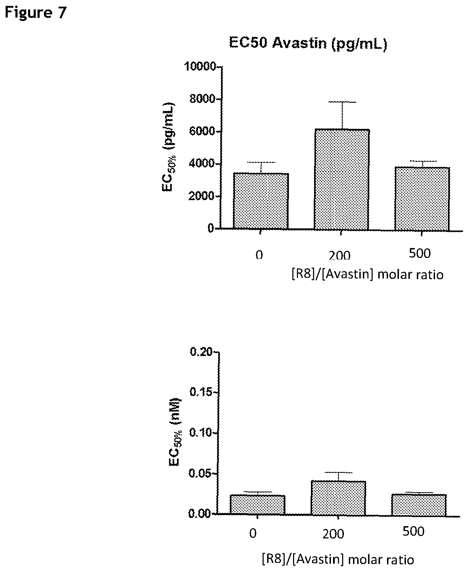

**Figure 7- Effect of R8 on VEGF$_{165}$ binding Avastin**

A functional ELISA was used to monitor the affinity of Avastin in the presence of varying concentrations of R8.

Addition of R8 to Avastin did not negatively impact VEGF binding activity.

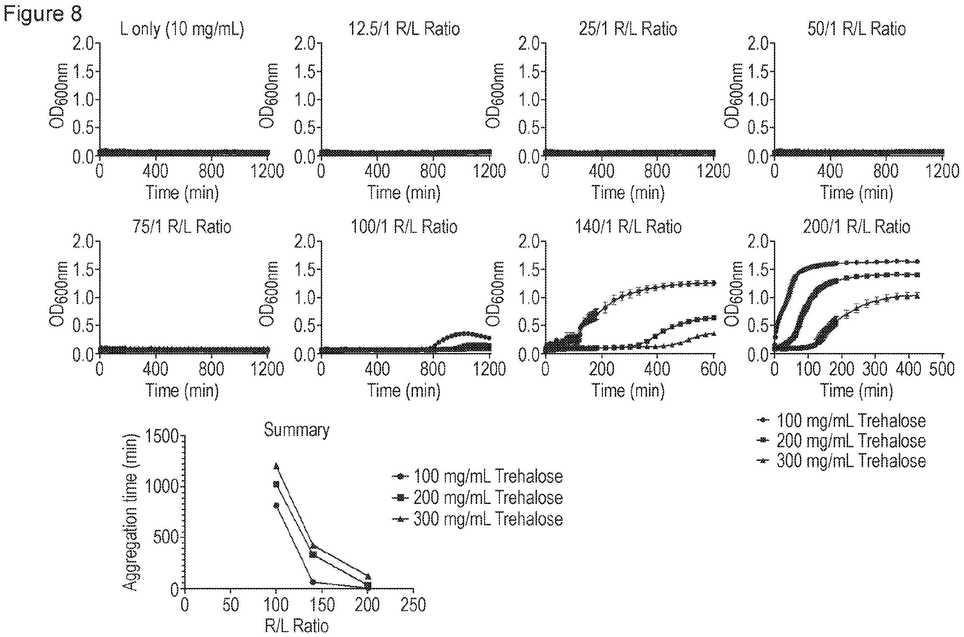

**Figure 8- Effect of R8:Lucentis ratio on stability at 25°C, and with the addition of Trehalose**

Absorbance (600nm) was used to assess R8:Lucentis (R/L) formulation aggregation over time.

The most stable formulations had a R/L ratio of 50 to 100/1.

Surprisingly, aggregation was delayed in the >100:1 R/L formulations by increasing trehalose concentration (100 to 300 mg/mL tested).

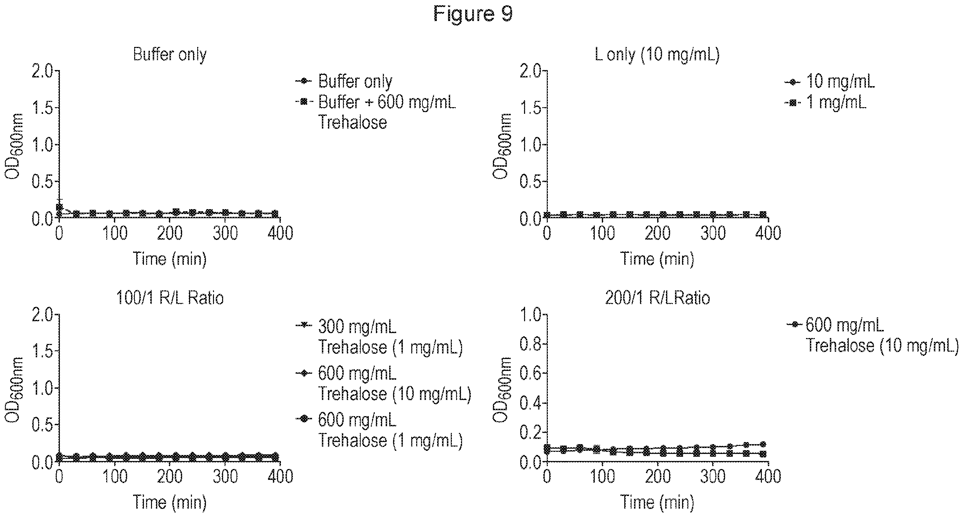

**Figure 9- Accelerated stability study (6 hours 25°C then 7 days 4°C)**

Increasing Trehalose (to 600 mg/mL) or reducing Lucentis concentration (1 mg/mL while maintaining R/L ratio) increased stability.

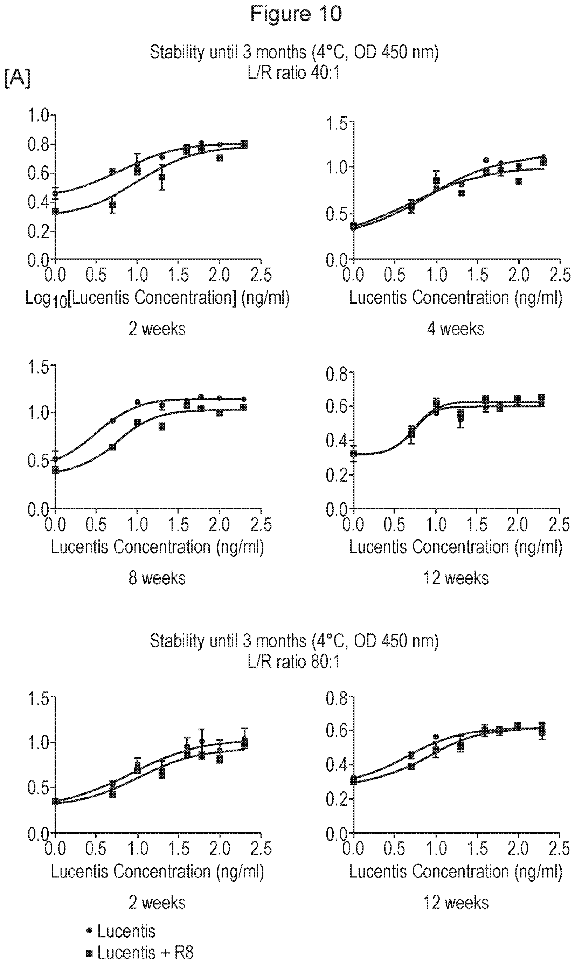

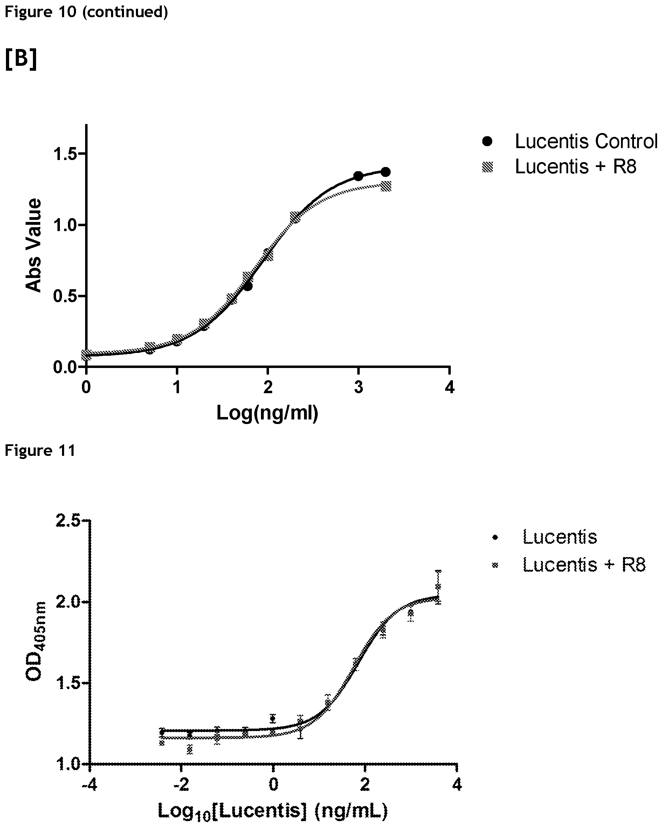

**Figure 10- R8/Lucentis is stable for 3 months at 4°C**

> **[A]** R8/Lucentis combinations at ratios of 40:1 and 80:1 are stable for 3 months at 4°C.
> **[B]** Stability of 12 weeks formulation at 4°C in 1:50 Lucentis:R8 (solution, not hydrogel). A Lucentis and R8 formulation 1:50 ratio combination remained stable after 12 weeks when stored at 4°C.

**Figure 11- ELISA detection of Lucentis for _in vivo_ work**

R8, though a highly charged peptide, does not interfere with the Lucentis detection ELISA used for the transwell assay.

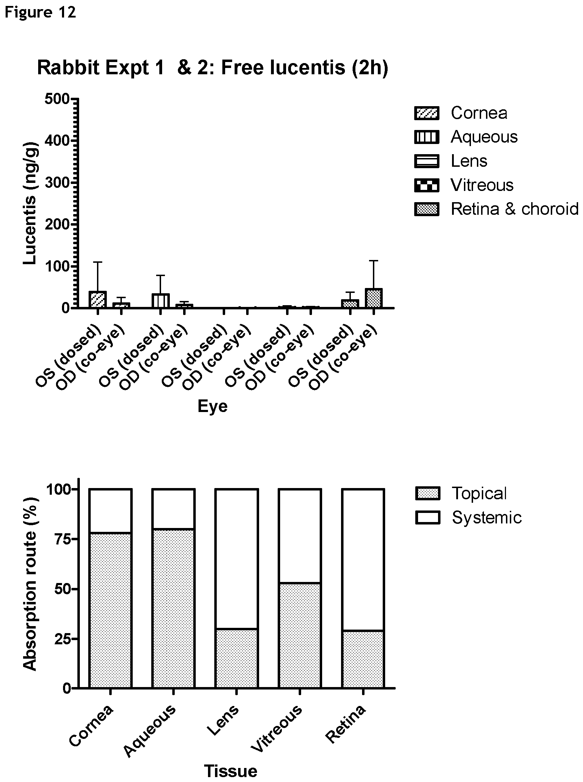

**Figure 12- Rabbit experiment: Free Lucentis (2h)**

Low levels (~20 ng) of free Lucentis were detected in retina 2h (previously established C$_{max}$) after topical instillation. This data was used as controls to compare with the formulation data. The concentration detected in plasma was minimal (<0.1 ng/mL). The proportion of topically and systemically delivered drug can be estimated using the following equation;

$$Topical\ absorbed\ (\%) = 100 * \frac{D}{(D + C)}$$

where D is the concentration of Lucentis detected in the dosed eye tissue and C is the concentration in the co-eye. The proportion of dosed free Lucentis reaching the retina by the topical route was ~30% (n=10).

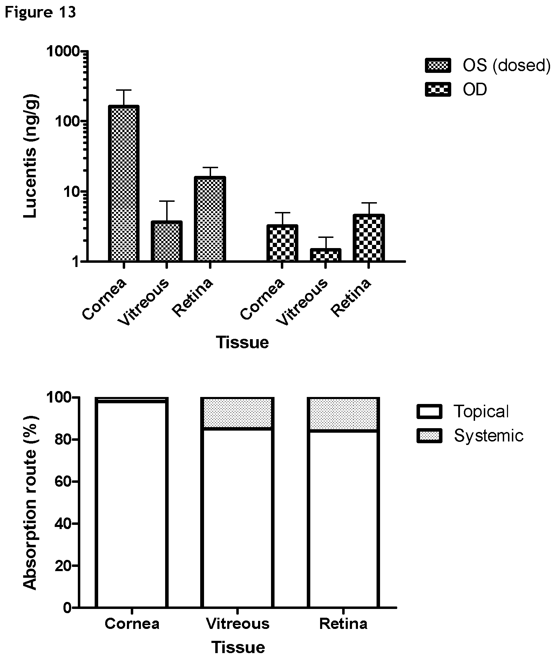

**Figure 13- R8:Lucentis at a ratio of 20/1 does not enhance delivery to retina after 2h**

100 mg/mL Lucentis with 50 mg/mL R8, no significant improvement in Lucentis delivery to the retina compared to Lucentis only control (n=3) (16+/- 6ng/g versus 18+/- 8ng/g). This ratio of R peptide to anti-VEGF to is similar to that reported in the de Cogan (2017) reference (25:1).

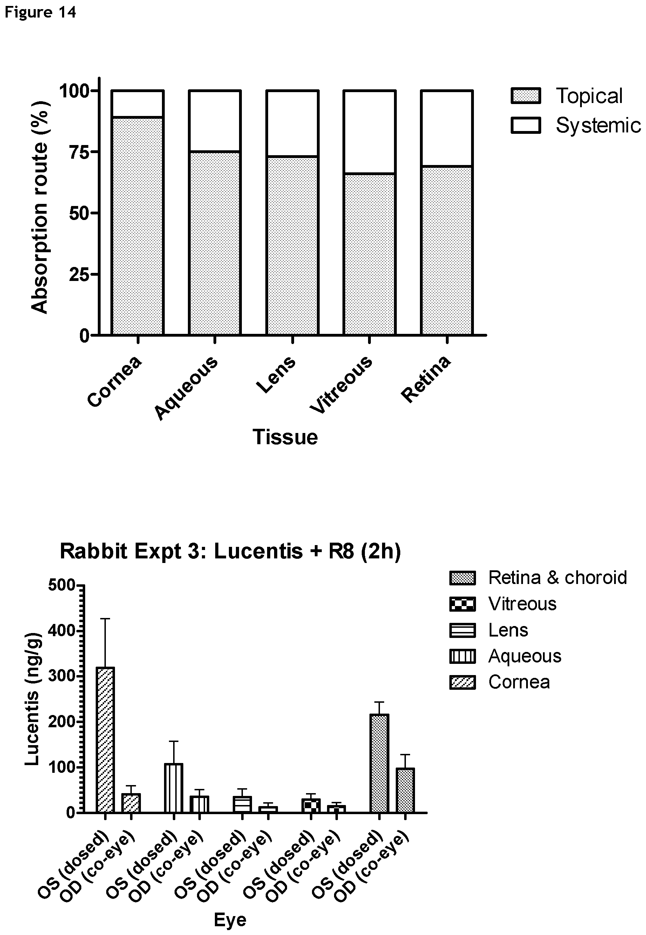

**Figure 14- R8: Lucentis at a ratio of 200/1 enhances delivery to retina after 2h** 10 mg/mL Lucentis with 50 mg/mL R8, significant improvement in Lucentis delivery to the retina compared to Lucentis only control.

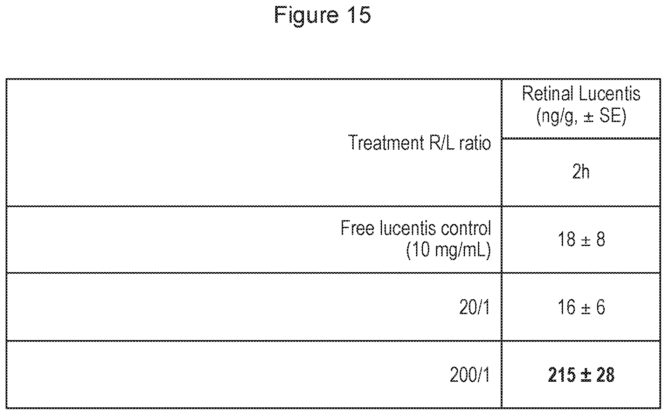

**Figure 15- Summary of Rabbit experiments**

_In vivo_ assessment of Lucentis concentrations in the retina after topical application of R/L formulations at 2 h. No significant difference in retinal Lucentis level when free Lucentis control is compared with 20/1 R/L ratio group. However, there is a significant increase ($\times$13.5 fold) in retinal level when 200/1 R/L ratio group is compared with 20/1 R/L group.

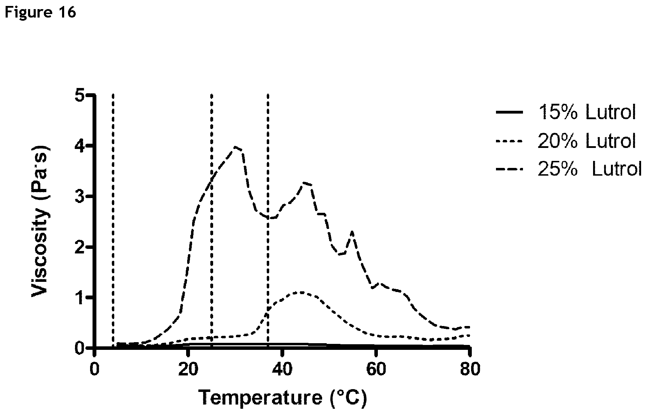

**Figure 16- Preparation of R8:Lucentis hydrogels to enhance delivery** To increase corneal contact time, R8:Lucentis formulations were made in with hydrogels to enhance formulation viscosity.

Lutrol F127 was chosen as it is already used in several existing clinical applications due to its thermosensitive properties.

The range of Lutrol F127 concentrations was between 15%-25% (w/v). Solutions were prepared by mixing the desired quantities of Lutrol F127. Between 20% and 25% Lutrol F127 was found to generate a thermosensitive gel with the desired properties.

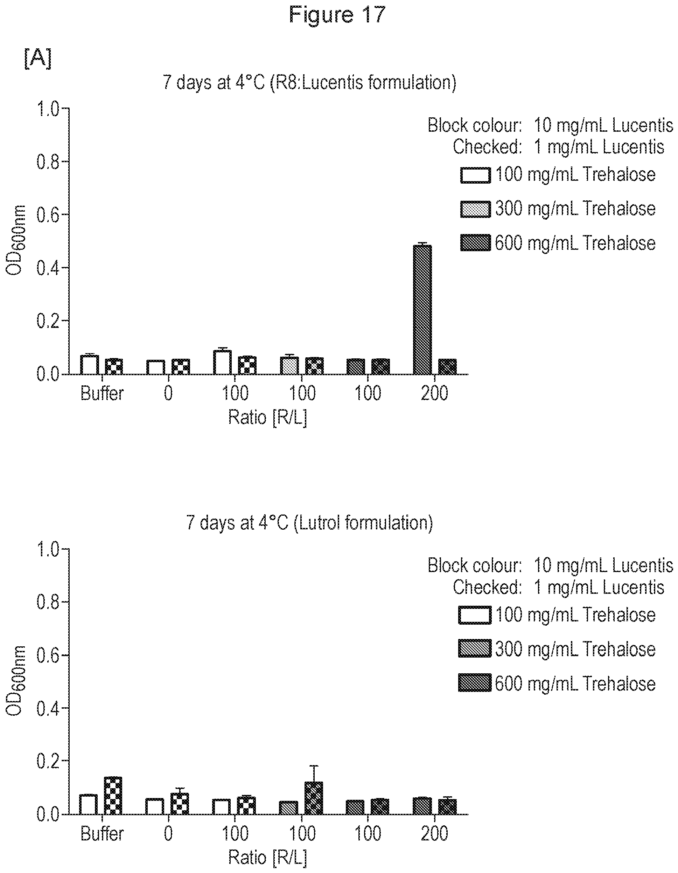

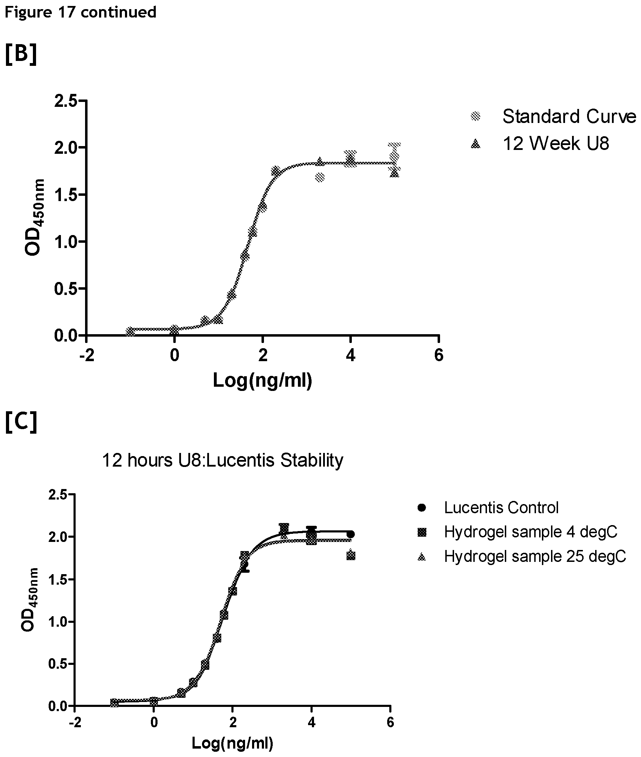

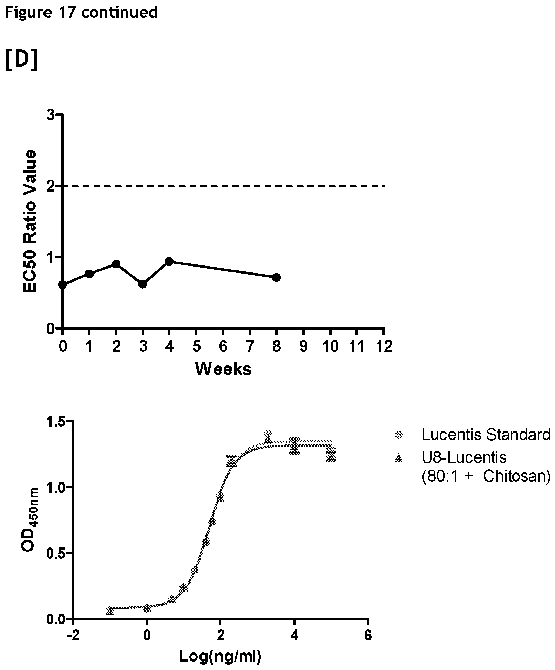

**Figure 17- Accelerated stability study (25°C then 4°C)**

> **[A]** 20% Lutrol hydrogel improves stability of Lucentis formulations. The presence of Lutrol helps prevent aggregation of R8:Lucentis formulations (as seen by increased OD) even at 200:1 ratio.
> **[B]** 12 week old formulation of U8:Lucentis Hydrogel showed good stability when stored at 4°C (1:50 Lucentis:R8 ratio).

[C] Accelerated stability testing of U8:Lucentis (50:1 ratio) 12h at 25°C showed no significant loss of VEGF binding activity (functional ELISA) compared to U8:Lucentis stored at 4°C or a Lucentis only control.

[D] Stability of chitosan-containing U8:Lucentis formulations at the 4 week time-point. Stability of the U8:Lucentis (80:1) formulation with 0.1% w/v chitosan showed good stability for 4 weeks as determined by $EC_{50}$ ratio (U8:Lucentis / Standard) of less than 2.

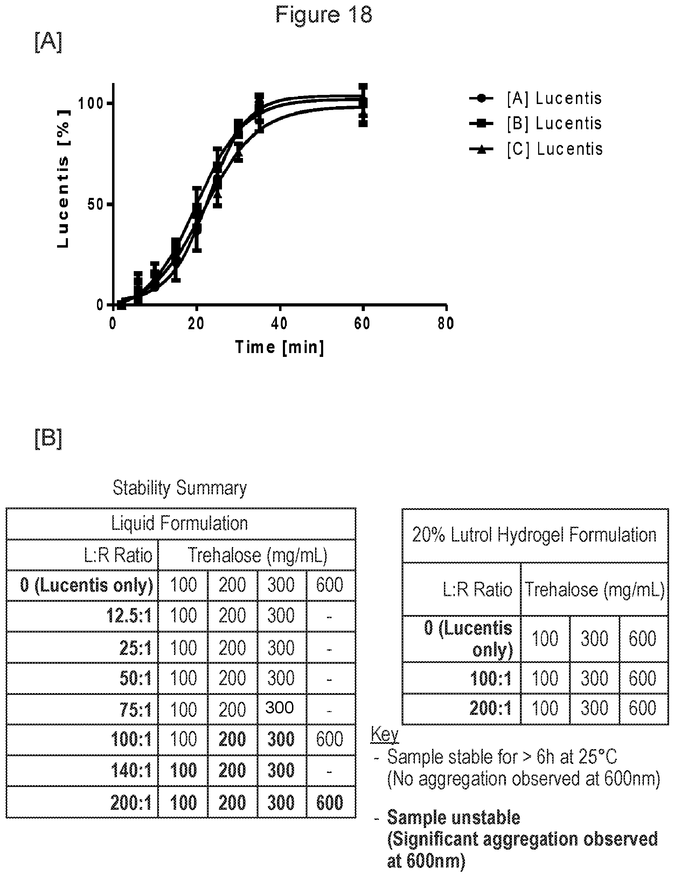

**Figure 18- Sustained release was proven *in vitro* using a Lucentis hydrogel release assay**

[A] (20% Lutrol gel) takes ~1h to liberate Lucentis. Similar results were obtained *in vivo* (Rabbit) with FITC loaded Lutrol hydrogels.

[B] Stability summary of R8/Lucentis liquid and hydrogel formulations

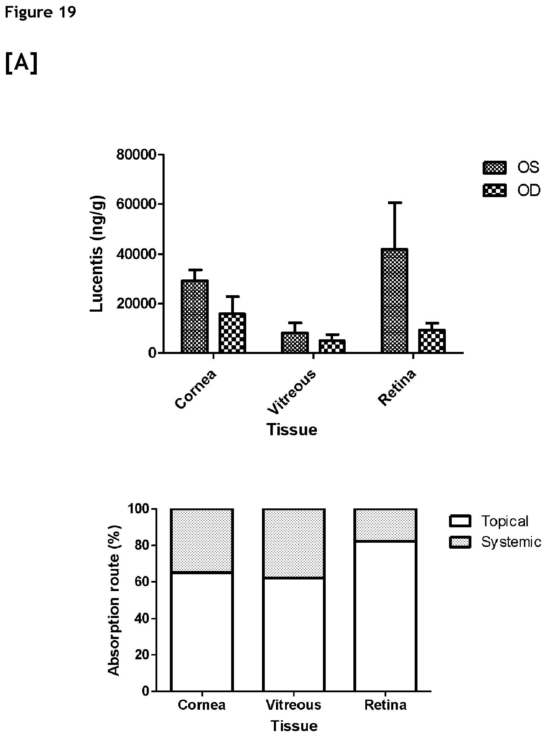

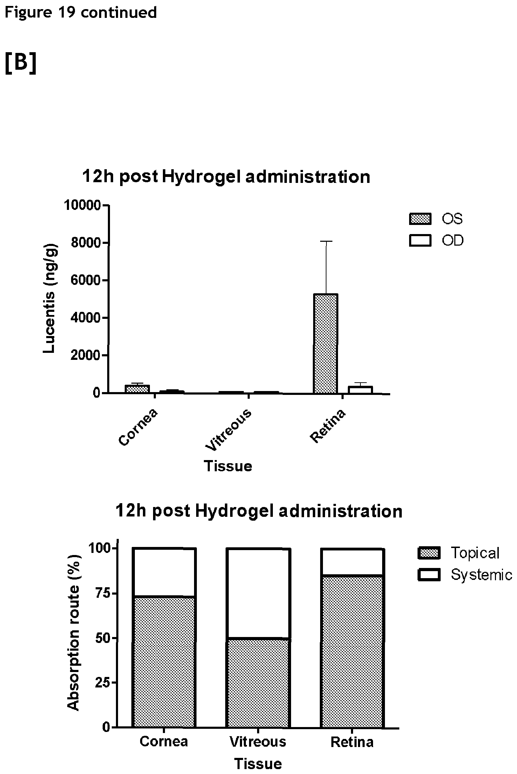

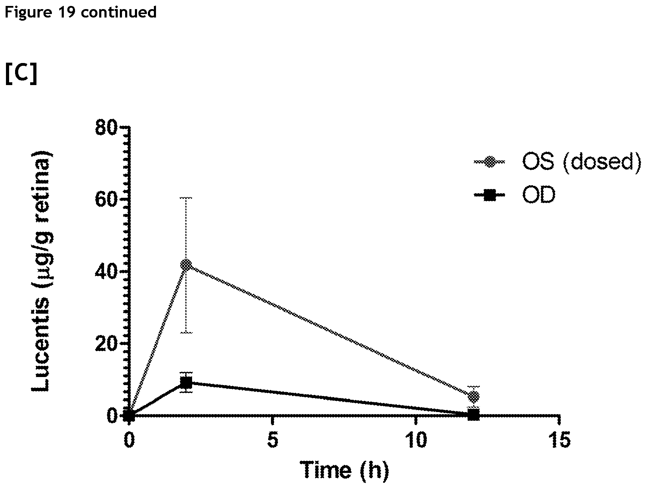

**Figure 19- Formulation of R8/Lucentis hydrogel 200:1 ratio (20% Lutrol) dramatically enhances delivery to retina**

[A] Lucentis gel 2h (200:1 ratio), n=3, Lucentis 10mg/ml and R8 50mg/ml. Significant concentrations of Lucentis were delivered to the retina vs undosed eye. Concentration reached 41823+/- 18709 ng/g retinal tissue, exceeding target of 200ng/g.

[B] Lucentis gel 12h (200:1 ratio), n=3, Lucentis 10mg/ml and R8 50mg/ml. Significant concentrations of Lucentis were delivered to the retina vs undosed eye. Concentration reached 5282+/- 2840 ng/g retinal tissue, exceeding target of 200ng/g.

[C] 200:1 R8: Lucentis hydrogel formulation summary of retinal delivery over time.

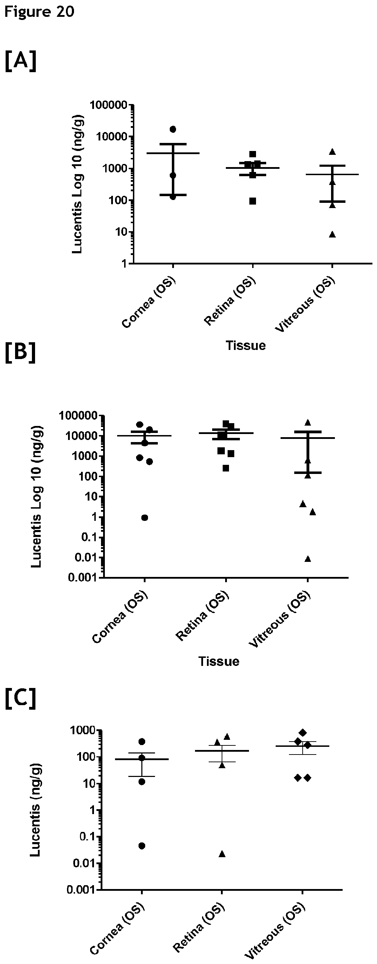

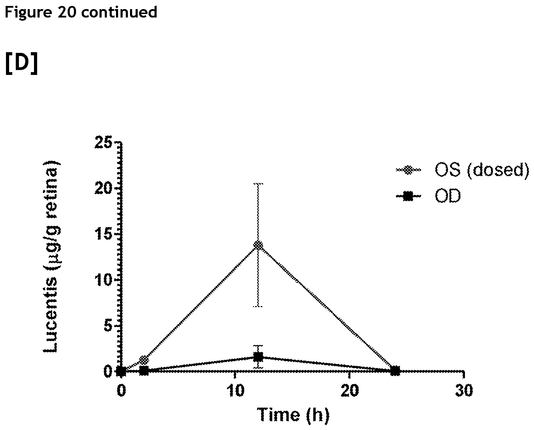

**Figure 20- Formulation of R8/Lucentis hydrogel 50:1 ratio (20% Lutrol) dramatically enhances delivery to retina**

[A] Lucentis gel 2h (50:1 ratio), n=6, Lucentis 10mg/ml and R8 12.5mg/ml. Significant concentrations of Lucentis were delivered to the retina vs undosed eye. Concentration reached 1254+/- 460 ng/g retinal tissue, exceeding target of 200ng/g.

[B] Lucentis gel 2h (50:1 ratio), n=6, Lucentis 10mg/ml and R8 12.5mg/ml. Significant concentrations of Lucentis were delivered to the retina vs undosed eye. Concentration reached 13790+/- 6725 ng/g retinal tissue, exceeding target of 200ng/g.

[C] Lucentis gel 2h (50:1 ratio), n=6, Lucentis 10mg/ml and R8 12.5mg/ml. Significant concentrations of Lucentis were delivered to the retina vs undosed eye. Concentration reached 169+/- 140 ng/g retinal tissue, below target of 200ng/g.

[D] 50:1 R8: Lucentis hydrogel formulation summary of retinal delivery over time.

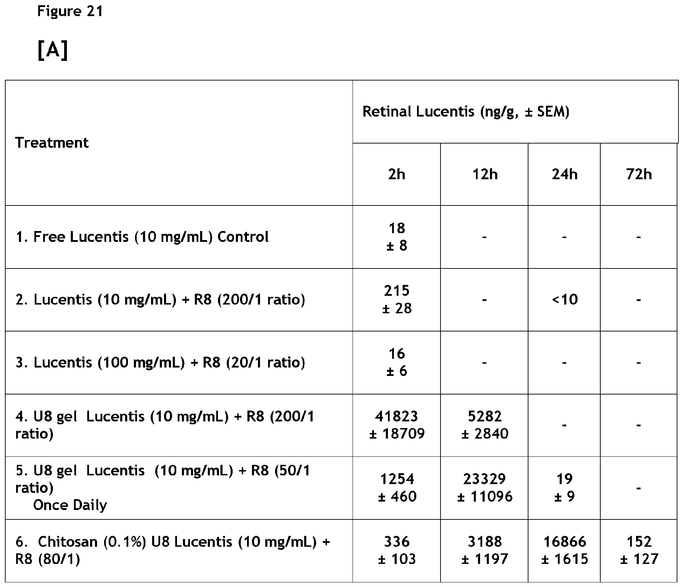

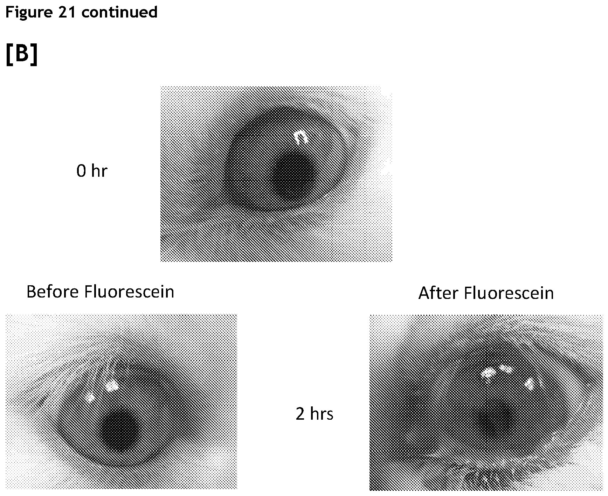

**Figure 21- Summary of hydrogel work and tolerability and toxicity of hydrogel formulation**

[A] *In vivo* assessment of Lucentis concentrations in the retina after topical application of R/L formulations at 2h, 12h, 24h and 72h. No significant difference in retinal Lucentis level when free Lucentis control was compared with 20/1 R/L ratio group at 2h. However, there was a significant increase (x13.5 fold) in retinal level when 200/1 R/L ratio group was compared with 20/1 R/L group at 2h. Furthermore, there was a ×195 fold increase retinal Lucentis level at 2h, retained at 12h, when the 200/1 Hydrogel formulation was compared with the 200/1 ratio group. There was also a significant increase in retinal level when 50/1 R/L ratio group was compared with 20/1 R/L group. In addition, further improvements in retinal delivery and retention were seen by increasing the R/L ratio to 80:1 and adding the mucoadhesive chitosan. An increase in the R/L ratio to 80:1 and the addition of a mucoadhesive (0.1% chitosan) were found to substantially improve delivery versus previous iterations.

[B] Rabbit eyes corneal toxicity assessment. Looking for evidence of redness, swelling, discharge, ulceration, haemorrhaging or scarring in the treated eye. There was no evidence of corneal toxicity or evidence of redness or corneal damage associated with U8:Lucentis administration (as seen by fluorescein staining).

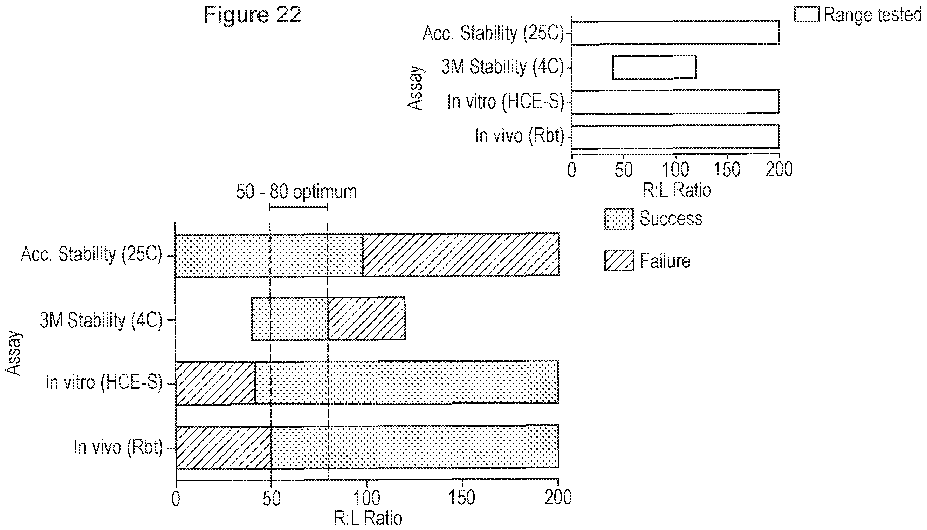

**Figure 22- Optimisation of formulation**

Formulations were tested in accelerated stability assays at 25°C, 3 month stability assays at 4°C, in the HCE-S transwell *in vitro* assay and in rabbit studies *in vivo.* A ratio range of R8 to Lucentis of 50-80:1 was found to be optimal.

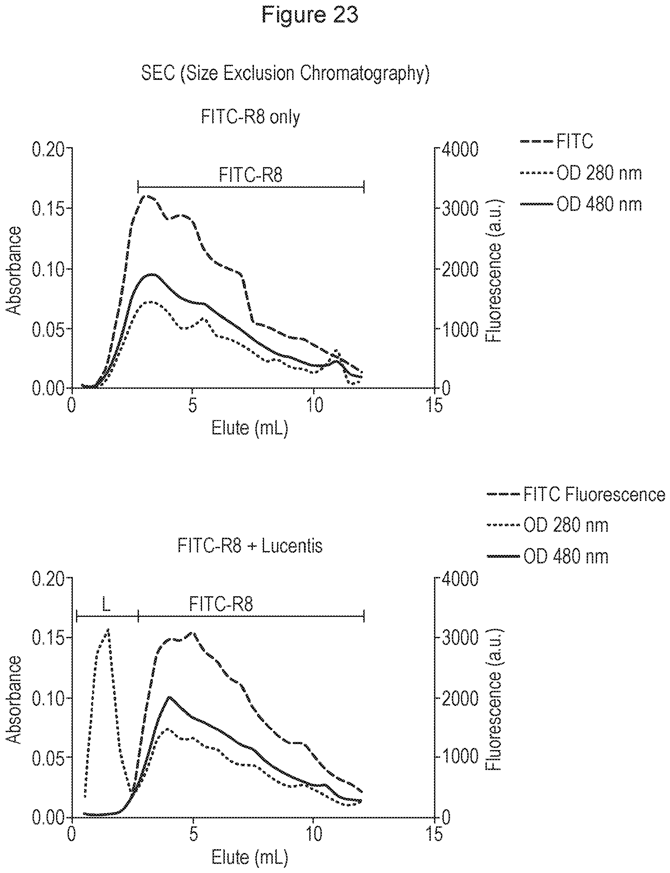

**Figure 23-Size Exclusion Chromatography to investigate binding of R8 to Lucentis** This chromatographic method is used for separation of molecules by size, or in some cases molecular weight. It is usually applied to large molecules or macromolecular complexes. There was no co-elution of Lucentis and FITC-R8, so no binding.

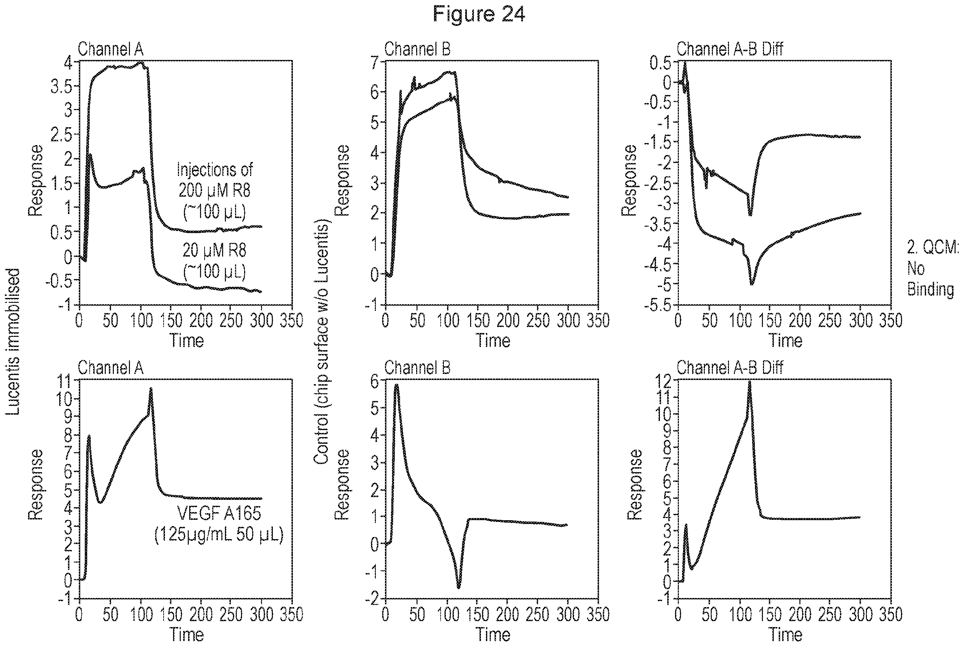

**Figure 24- Quartz Crystal Microbalance to investigate binding of R8 to Lucentis** This method measures a mass variation per unit area by measuring the change in frequency of a quartz crystal resonator. The resonance is disturbed by the addition or removal of a small mass due to oxide growth/decay or film deposition at the surface of the acoustic resonator. 10 μg/ml Lucentis used, approx. 100 μl injections. No binding to R8 was detected.

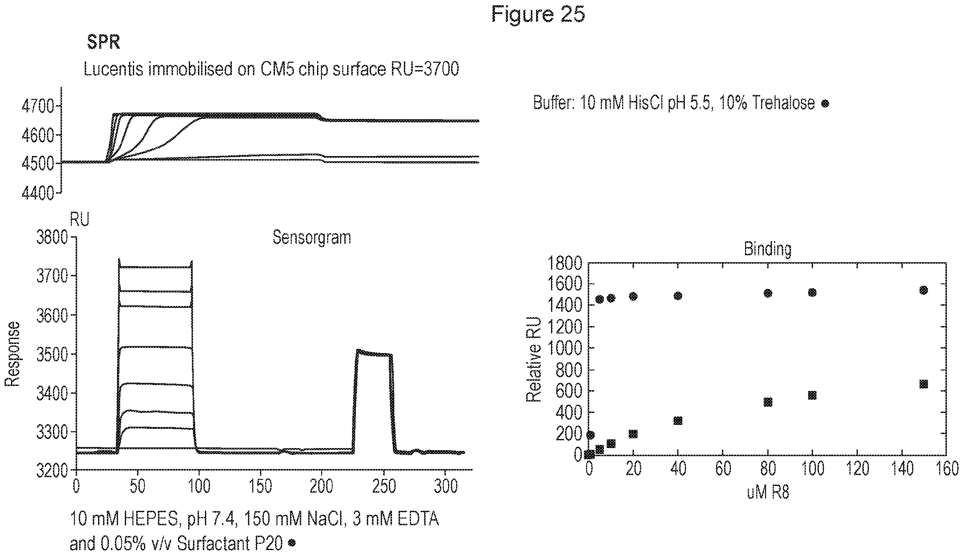

**Figure 25- Surface Plasmon Resonance to investigate binding of R8 to Lucentis** Lucentis immobilised on CM5 chip surface RU = 3700. Some binding of R8 to Lucentis was detected.

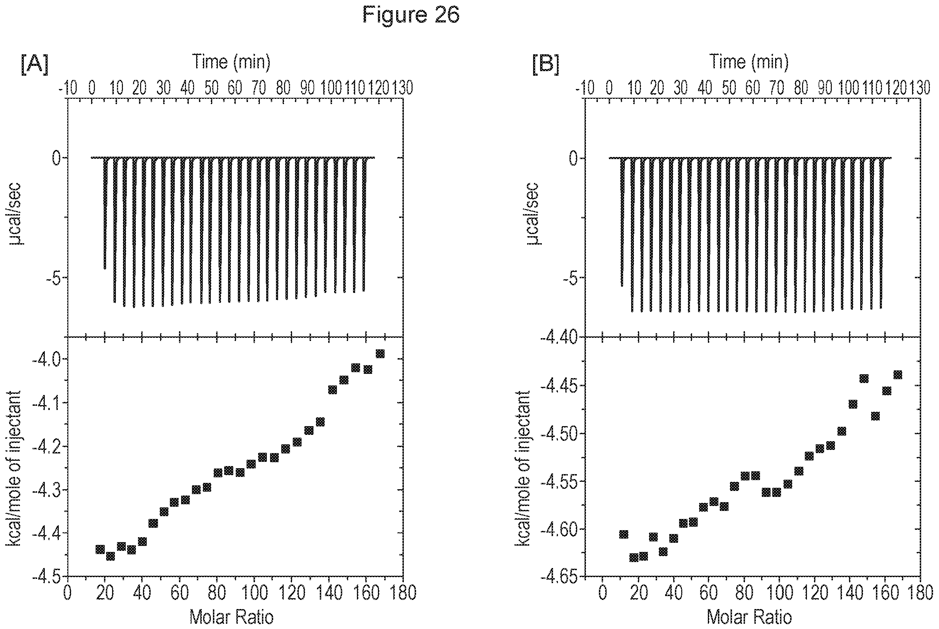

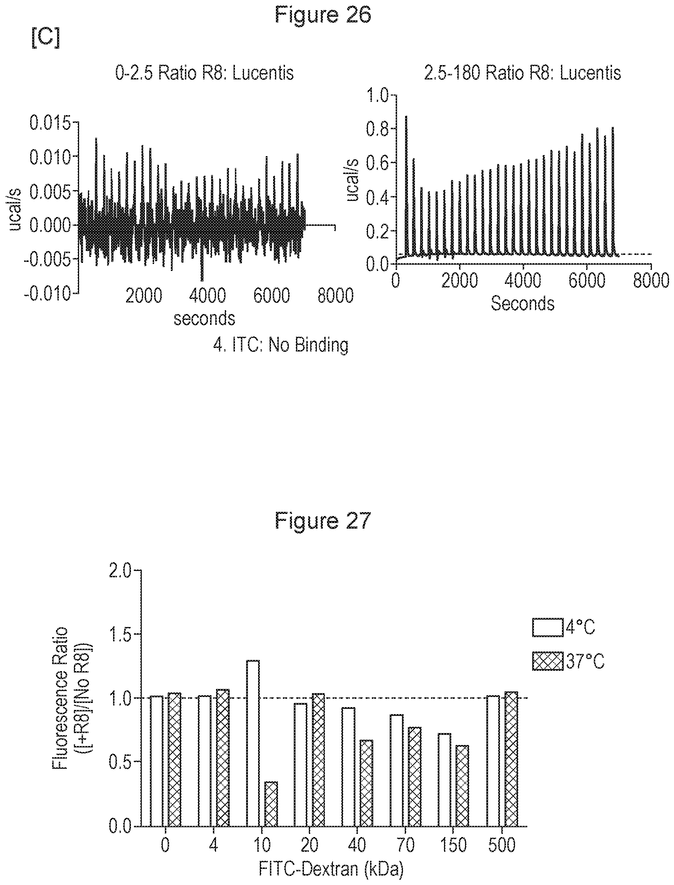

**Figure 26- Isothermal titration calorimetry (ITC) to investigate binding of R8 to Lucentis**
10mM HisCl, 10% Trehalose, pH 5.5

**[A]** R8:Lucentis molar ratio
**[B]** R8 heats of dilution (ratios plotted just for illustration purposes)
**[C]** ITC results with 0-2.5 ratio of R8:Lucentis and 2.5-180 ratio of R8:Lucentis. No binding was detected.

**Figure 27- The transport of dextrans used to investigate whether R8 can carry dextrans across the membrane** R8 made no difference to the transport of different sizes of dextran across a membrane.

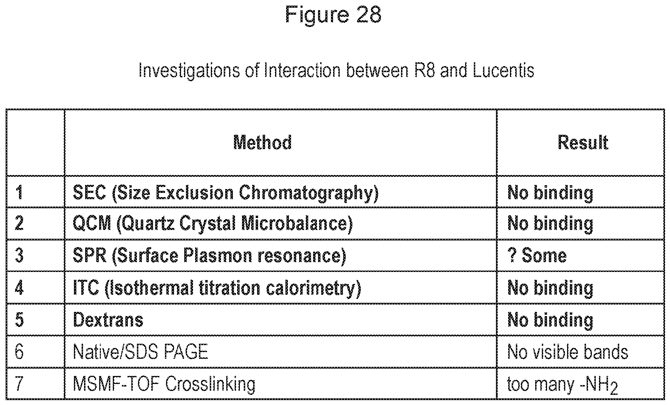

**Figure 28** - **Summary of investigations of interaction between R8 and Lucentis**

## DETAILED DESCRIPTION OF THE INVENTION

It is to be understood that different applications of the disclosed methods may be tailored to the specific needs in the art. It is also to be understood that the terminology used herein is for the purpose of describing particular embodiments of the invention only, and is not intended to be limiting.

In addition as used in this specification and the appended claims, the singular forms "a", "an", and "the" include plural references unless the content clearly dictates otherwise. Thus, for example, reference to "small cationic peptide" includes "small cationic peptides", and the like. "R8: Lucentis", "R8/Lucentis" or "R8 to Lucentis" are used interchangeably to mean the ratio of R8 to Lucentis.

The terms "U8 Lucentis", "U8 gel Lucentis" and "U8 Lucentis Hydrogel" are used interchangeably to mean a formulation of R8 and Lucentis that contains a hydrogel and trehalose.

The composition of the invention comprises Ranibizumab (Lucentis) and the small cationic peptide R8, RRRRRRRR, (SEQ ID NO: 1).

### VEGF antagonist

VEGF antagonists can be defined as any agent that prevents or inhibits the expression or function of VEGF. VEGF antagonists can be proteins. VEGF antagonists can be a soluble receptor of VEGF, such as Aflibercept (Eylea), or an anti-VEGF antibody. VEGF antagonists can be diabodies or bispecific antibodies. VEGF antagonists can be fragments or derivatives of anti-VEGF antibodies. Examples of antibody fragments or derivatives include a Fab fragment, a F(ab')$_2$ fragment, a Fab' fragment, a Fd fragment, a Fv fragment, a dAb fragment and an isolated complementarity determining region (CDR). Single chain antibodies such as scFv antibodies are also intended to be encompassed. Thus VEGF antagonists can be scFv antibodies. The VEGF antagonist of the invention is the anti-VEGF antibody Ranibizumab (Lucentis).

### Small cationic peptide

Small cationic peptides from part of the composition of the invention. Small cationic peptides of the invention are cell penetrating peptides (CPP). Small cationic peptides of the invention aid the uptake of substances into tissues. Small cationic peptides of the invention aid the intracellular update of substances. Small cationic peptides of the invention enhance the transcytosis of substances. Intracellular update and transcytosis can be measured by the skilled person by methods known in the art. For example, transcytosis can be measured by an *in vitro* HCE-S transcytosis assay (as described in Davis et al (2014)).

Small cationic peptides may consist of 6, 7, 8, 9, 10, 11, 12, 13, 14, 15, 16, 17, 18, 19 or 20 amino acids and have an overall positive charge due to the presence of cationic amino acids. Cationic amino acids have a positive charge at physiological pH. Examples of such positively charged amino acids include Lysine, Histidine and Arginine.

It will be understood that small cationic peptides of the invention are intended to encompass certain variants of said peptides. Other than the engineering to reduce dimer formation discussed above, other modifications to the native amino acid sequence of a peptide may be made. For example to one, two, three, four, or five amino acids in the sequence of a peptide. Any such modifications will typically be conservative, in that if an amino acid in the native sequence is replaced with a different amino acid, the different amino acid will typically have similar properties to the native amino acid. The table below shows the properties of amino acids. Molecular weights are shown alongside the 3-letter code for each amino acid. The molecular weights given are those of the neutral, free amino acids; residue weights can be obtained by subtraction of one equivalent of water (18 g/mol). The invention also includes peptides containing N- and C-terminals modified or blocked to reduce or inhibit degradation by exopeptidase enzymes.

| Ala | 89 | Aliphatic, hydrophobic, neutral | Met | 149 | hydrophobic, neutral |
|-----|-----|-----|-----|-----|-----|
| Cys | 121 | polar, hydrophobic, neutral | Asn | 132 | polar, hydrophilic, neutral |
| Asp | 133 | polar, hydrophilic, charged (-) | Pro | 115 | hydrophobic, neutral |
| Glu | 147 | polar, hydrophilic, charged (-) | Gln | 146 | polar, hydrophilic, neutral |
| Phe | 165 | Aromatic, hydrophobic, neutral | Arg | 174 | polar, hydrophilic, charged (+) |
| Gly | 75 | Aliphatic, neutral | Ser | 105 | polar, hydrophilic, neutral |
| His | 155 | aromatic, polar, hydrophilic, charged (+) | Thr | 119 | polar, hydrophilic, neutral |
| Ile | 131 | Aliphatic, hydrophobic, neutral | Val | 117 | aliphatic, hydrophobic, neutral |
| Lys | 146 | polar, hydrophilic, charged(+) | Trp | 204 | aromatic, hydrophobic, neutral |
| Leu | 131 | Aliphatic, hydrophobic, neutral | Tyr | 181 | aromatic, polar, hydrophobic |

The residue or residues which are modified may be comprised in any part of the sequence.

Small cationic peptides of the invention aid the uptake of VEGF antagonists into tissues. These peptides aid the uptake of VEGF antagonists into the retina when the composition of the invention is topically administered to the eye. These peptides aid the transcytosis of VEGF antagonists to reach the retina when the composition of the invention is topically administered to the eye. In the invention the small cationic peptide is an R8 peptide, or a modified R8 peptide (e.g. Stearyl-R8 (ST-R8)). R8 is defined as being eight consecutive arginine resides, RRRRRRRR (SEQ ID NO:1).

In the composition of the invention, the ratio of small cationic peptide to VEGF antagonist is set out as a molar ratio. In the composition of the invention, the ratio of R8 to Lucentis is at least 40:1, at least 50:1, at least 60:1, at least 70:1, at least 80:1, at least 90:1, at least 100:1, at least 150:1 or at least 200:1. A preferred ratio range of R8 to Lucentis is 50:1 to 80:1.

**Peptide Synthesis**

The small cationic peptides of the invention may be synthesised using methods well known in the art. Preferred methods include solid-phase peptide synthesis techniques and most preferably an automated or semiautomated peptide synthesizer. Typically, using such techniques, an $\alpha$-N-carbamoyl protected amino acid and an amino acid attached to the growing peptide chain on a resin are coupled at room temperature in an inert solvent such as dimethylformamide, N-methylpyrrolidinone or methylene chloride in the presence of coupling agents such as dicyclohexylcarbodiimide and 1-hydroxybenzotriazole in the presence of a base such as diisopropyl-ethylamine. The $\alpha$-N-carbamoyl protecting group is removed from the resulting peptide-resin using a reagent such as trifluoroacetic acid or piperidine, and the coupling reaction repeated with the next desired N-protected amino acid to be added to the peptide chain. Suitable N-protecting groups are well known in the art, and include t-butyloxycarbonyl (tBoc) and fluorenylmethoxycarbonyl (Fmoc).

The term "peptide" includes not only molecules in which amino acid residues are joined by peptide (-CO-NH-) linkages but also molecules in which the peptide bond is reversed. Such retro-inverse peptidomimetics may be made using methods known in the art, for example such as those described in Meziere et al (1997) J. Immunol.159, 3230-3237. This approach involves making pseudopeptides containing changes involving the backbone, and not the orientation of side chains. Retro-inverse peptides, which contain NH-CO bonds instead of CO-NH peptide bonds, are much more resistant to proteolysis.

Similarly, the peptide bond may be dispensed with altogether provided that an appropriate linker moiety which retains the spacing between the carbon atoms of the amino acid residues is used; it is particularly preferred if the linker moiety has substantially the same charge distribution and substantially the same planarity as a peptide bond. It will also be appreciated that the peptide may conveniently be blocked at its N-or C-terminus so as to help reduce susceptibility to exoproteolytic digestion. For example, the N-terminal amino group of the peptides may be protected by reacting with a carboxylic acid and the C-terminal carboxyl group of the peptide may be protected by reacting with an amine. Other examples of modifications include glycosylation and phosphorylation. Another potential modification is that hydrogens on the side chain amines of R or K may be replaced with methylene groups ($-NH_2$ replaced with $-NH(Me)$ or $-N(Me)_2$) (methylation). Another potential modification is that hydrogens of a hydroxyl group of an amino acid such as K can be replaced with an acetyl group (CH3CO) (acetylation).

Analogues of peptides according to the invention may also include peptide variants that increase or decrease the peptide's half-life *in vivo*. Examples of analogues capable of increasing the half-life of peptides used according to the invention include peptoid analogues of the peptides, D-amino acid derivatives of the peptides, and peptide-peptoid hybrids. A further embodiment of the variant polypeptides used according to the invention comprises D-amino acid forms of the polypeptide. The preparation of polypeptides using D-amino acids rather than L-amino acids greatly decreases any unwanted breakdown of such an agent by normal metabolic processes, decreasing the amounts of agent which needs to be administered, along with the frequency of its administration.

**Formulations**

In a preferred embodiment, the composition of the invention may also comprise other components. Such components may act to improve the stability of the formulation, by for example preventing aggregation, or may act to improve its efficacy. Such components may act to improve the stability of the formulation so that it is generally more stable during storage and transport and has a longer shelf-life than the equivalent formulation without the additional component.

In a preferred embodiment of the invention the composition of the invention also comprises Trehalose. Trehalose acts to delay aggregation of the R8 and Ranibizumab formulation. The concentration of Trehalose in the composition can be 100mg/ml or more, 200mg/ml or more, 300mg/ml or more, 400mg/ml or more, 500 mg/ml or more or preferably 600 mg/ml or more. In a preferred embodiment of the invention the composition of the invention also comprises Trehalose at a concentration of approximately 100mg/ml.

Other alternative or additional agents which can act to improve the stability of compositions include trehalose sugar, buffers, tonicity agents, simple carbohydrates, sugars such as sorbitol or sucrose, , carbohydrate polymers, amino acids, oligopeptides, polyamino acids, polyhydric alcohols and ethers thereof, detergents, lipids, surfactants, antioxidants, salts, human serum albumin, gelatins, formaldehyde, polysorbate 80 or combinations thereof.

In a preferred embodiment of the invention, the composition of the invention comprises an agent that increases the viscosity of the composition. Viscosity can be measure by the skilled person by methods known in the art, for example by using a viscometer or rheometer. An example of an agent that increases viscosity is a hydrogel. In a preferred embodiment of the invention, the composition of the invention comprises a hydrogel. The hydrogel improves the contact time of the composition with the tissue of interest, for example the cornea, by increasing the viscosity of the composition. Common ingredients in hydrogels include polyvinyl alcohol, sodium polyacrylate, acrylate polymers and copolymers with an abundance of hydrophilic groups. Natural hydrogel materials are being investigated for tissue engineering; these materials include agarose, methylcellulose, hyaluronan, and other naturally derived polymers.

Alternative agents that can be added to the composition to increase the viscosity of the composition include Lutrol (Poloxamer) Chitosan, gellants, polysaccharides, proteins, polyethylene glycol and carbomers.

The hydrogel concentration in the composition of the invention may be between 15% and 25% w/v. Preferably, the concentration of hydrogel is between 20% and 25% w/v. The hydrogel can be, for example, hyaluronic acid, hypermellose, or another biocompatible hydrogel gel-forming material. In a preferred embodiment the hydrogel is Lutrol F127.

In a preferred embodiment, the composition of the invention comprises both Trehalose and a hydrogel. In a preferred embodiment, the composition of the invention comprises R8 and Lucentis at a ratio of at least 40:1, Trehalose and a hydrogel. In a preferred embodiment, the composition of the invention comprises R8 and Lucentis at a ratio of at least 50:1, Trehalose and a hydrogel. In a preferred embodiment, the composition of the invention comprises R8 and Lucentis at a ratio of 50:1 to 80:1, Trehalose and a hydrogel. In a preferred embodiment, the composition of the invention comprises R8 and Lucentis at a ratio of at least 100:1, Trehalose and a hydrogel. In a preferred embodiment, the composition of the invention comprises R8 and Lucentis at a ratio of at least 200:1, Trehalose and a hydrogel.

In a preferred embodiment, the composition of the invention comprises R8 and Lucentis at a ratio of at least 40:1, Trehalose at a concentration of at least 100mg/ml and a hydrogel. In a preferred embodiment, the composition of the invention comprises R8 and Lucentis at a ratio of at least 50:1, Trehalose at a concentration of at least 100mg/ml and a hydrogel. In a preferred embodiment, the composition of the invention comprises R8 and Lucentis at a ratio of 50:1 to 80:1, Trehalose at a concentration of at least 100mg/ml and a hydrogel.

In a preferred embodiment, the composition of the invention comprises R8 and Lucentis at a ratio of at least 40:1, Trehalose at a concentration of at least 200mg/ml and a hydrogel. In a preferred embodiment, the composition of the invention comprises R8 and Lucentis at a ratio of at least 50:1, Trehalose at a concentration of at least 200mg/ml and a hydrogel. In a preferred embodiment, the composition of the invention comprises R8 and Lucentis at a ratio of 50:1 to 80:1, Trehalose at a concentration of at least 200mg/ml and a hydrogel.

In a preferred embodiment, the composition of the invention comprises R8 and Lucentis at a ratio of at least 40:1, Trehalose at a concentration of at least 300mg/ml and a hydrogel. In a preferred embodiment, the composition of the invention comprises R8 and Lucentis at a ratio of at least 50:1, Trehalose at a concentration of at least 300mg/ml and a hydrogel. In a preferred embodiment, the composition of the invention comprises R8 and Lucentis at a ratio of 50:1 to 80:1, Trehalose at a concentration of at least 300mg/ml and a hydrogel.

In a preferred embodiment, the composition of the invention comprises R8 and Lucentis at a ratio of at least 40:1, Trehalose at a concentration of at least 600mg/ml and a hydrogel. In a preferred embodiment, the composition of the invention comprises R8 and Lucentis at a ratio of at least 200:1, Trehalose at a concentration of at least 600mg/ml and a hydrogel.

In a preferred embodiment, the composition of the invention comprises R8 and Lucentis at a ratio of at least 40:1, Trehalose and a hydrogel at a concentration of at least 15%-25% w/v. In a preferred embodiment, the composition of the invention comprises R8 and Lucentis at a ratio of at least 40:1, Trehalose and a hydrogel at a concentration of at least 20%-25% w/v.

In a preferred embodiment, the composition of the invention comprises R8 and Lucentis at a ratio of at least 50:1, Trehalose and a hydrogel at a concentration of at least 20%-25% w/v. In a preferred embodiment, the composition of the invention comprises R8 and Lucentis at a ratio of 50:1 to 80:1, Trehalose and a hydrogel at a concentration of at least 20%-25% w/v. In a preferred embodiment, the composition of the invention comprises R8 and Lucentis at a ratio of 50:1 to 80:1, Trehalose at a concentration of at least 100mg/ml and a hydrogel at a concentration of at least 20%-25% w/v.

In a preferred embodiment, the composition of the invention comprises R8 and Lucentis at a ratio of 50:1 to 80:1, Trehalose at a concentration of at least 200mg/ml and a hydrogel at a concentration of at least 20%-25% w/v. In a preferred embodiment, the composition of the invention comprises R8 and Lucentis at a ratio of at least 200:1, Trehalose at a concentration of at least 600mg/ml and a hydrogel at a concentration of at 20%-25% w/v.

An example of a final formulation of the invention comprises 8mg/ml Lucentis (0.173mM) (10mg/ml Lucentis, 10mM histidine HCL, 100mg/ml Trehalose, 0.01% polysorbate 80, pH 5.5), 10 mg/ml R8 (molar ratio R8:Lucentis 50:1), 200 mg/ml Trehalose, 20% w/v Lutrol-F127, 0.01% polysorbate 80, pH 5.5.

In a preferred embodiment, the composition of the invention also comprises a mucoadhesive polymer. Mucoadhesive polymers are already in clinical use to further enhance the delivery of Lucentis to posterior ocular tissues. An example of a suitable mucoadhesive polymer for use in the invention is chitosan. Whilst to date there is no current EU approved medicinal product which includes chitosan, there has been a clinical trial with chitosan, most notably in eye drops for dry eyes: Local Tolerability of Chitosan-N-acetylcysteine Eye Drops in Healthy Young Volunteers ClinicalTrials.gov Identifier: NCT01278784.

An eye drop containing chitosan has been previously available under the trade name Lacrimera by Croma Pharma for the treatment of dry eyes (Schmidl, D. et al., 2017). There is also literature to support the use of chitosan with a good safety and tolerability profile but in limited patient numbers (Fischak C., et al., 2017).

In a preferred embodiment, the composition of the invention comprises a small cationic peptide and a VEGF antagonist at a ratio of 50:1 to 80:1, a hydrogel and a mucoadhesive polymer. In a preferred embodiment, the composition of the invention comprises a small cationic peptide and a VEGF antagonist at a ratio of 50:1 to 80:1, a hydrogel and chitosan. Preferably, the small cationic peptide is R8. Preferably, the VEGF antagonist is Lucentis. Preferably, the small cationic peptide is R8 and the VEGF antagonist is Lucentis. Preferably, the chitosan is present at a concentration of between 0.05 % and 0.25% w/v. Preferably the ratio of R8 to Lucentis is 80:1. Preferably the ratio of R8 to Lucentis is 80:1 and the chitosan is present at a concentration of between 0.05% and 0.25% w/v. Preferably the ratio of R8 to Lucentis is 80:1 and chitosan is present at a concentration of 0.1 w/v. Preferably the ratio of R8 to Lucentis is 80:1, the hydrogel is present at a concentration of 20%-25% w/v and chitosan is present at a concentration of 0.1% w/v. Preferably the chitosan is 5k chitosan. Preferably the composition also comprises trehalose.

In a preferred embodiment, the composition of the invention comprises R8 and Lucentis at a ratio of 80:1, a hydrogel and chitosan. In a preferred embodiment, the composition of the invention comprises R8 and Lucentis at a ratio of 80:1, trehalose, a hydrogel and chitosan. In a preferred embodiment, the composition of the invention comprises R8 and Lucentis at a ratio of 80:1, trehalose at a concentration of 10% w/v, a hydrogel at a concentration of 20% w/v and chitosan at a concentration of 0.1% w/v. In a preferred embodiment, the composition of the invention comprises R8 and Lucentis at a ratio of 80:1, trehalose at a concentration of 10% w/v, a hydrogel at a concentration of 20% w/v and 5k chitosan at a concentration of 0.1% w/v.

An example of a final formulation of the invention comprises 10 mg/ml Lucentis, 20 mg/ml R8 (molar ratio R8:Lucentis 80:1), 20% w/v Lutrol-F 127, 10% w/v $\alpha,\alpha$-trehalose dehydrate, 0.1% w/v 5k chitosan, 10 mM histidine HCl, 0.01% w/v polysorbate 80, pH 5.5.

**Therapeutic indications**

The compositions of the present invention can be used to treat or prevent diseases or conditions characterised by VEGF dysfunction. The compositions of the present invention can be used to treat or prevent diseases or conditions where VEGF is functioning aberrantly. The compositions of the present invention can be used to treat diseases currently treated by an VEGF antagonist. The compositions of the present invention can be used to prevent or treat diseases or conditions characterised by VEGF dysfunction such as vasculoproliferative conditions, cancers, oedema or leaky vessels, retinal vein occlusions, diabetic vasculopathy, diseases characterised by retinal angiogenesis, sickle cell disease and retinopathy of prematurity.

The compositions of the present invention can be used to treat or prevent diseases or conditions characterised by VEGF dysfunction such as vasculoproliferative conditions. "Vasculoproliferation", "vasculoproliferative", "vasculoproliferative conditions" and similar terms as used herein encompass any and all pathologies related to the aberrant or unwanted development of blood vessels or vascular tissue or cells. For example, both pathogenic angiogenesis (the formation of new blood vessels, for example via new capillary growth from existing blood vessels) and vascular malformation (e.g. telangiectasia, the formation of dilated, tortuous and incompetent vessels, microaneurysms) can be prevented or reduced, as can neovascularisation and vascular endothelial cell proliferation. Also, as is known in the art, neoplastic growth requires the formation of new blood vessels to provide a blood supply to the growing tumour.

Tumours in which vasculoproliferation occurs are therefore also conditions which may be treated, prevented or ameliorated according to the present invention.

Treatment of ocular vasculoproliferative conditions is a preferred embodiment of the invention. Among conditions that can be treated are: age-related macular degeneration (AMD), diabetic retinopathy, retinal vein occlusion, retinopathy of prematurity, macular telangiectasia, or choroidal neovascularisation, diabetic vasculopathy, diseases characterised by retinal angiogenesis and sickle cell disease. Age-related macular degeneration can include "wet" or "dry" AMD.

Treatment of tumours, typically solid tumours, can also be effected, in that preventing angiogenesis in tumours derives the tumour of blood supply. Tumour treatment targets include brain, breast, kidney, colorectal, lung, prostate, head and neck, stomach, pancreatic, skin, cervical, bone, ovarian, testicular and liver tumours.

## Pharmaceutical Compositions, Dosages and Dosage Regimes

Compositions of the invention will typically be formulated into pharmaceutical compositions, together with a pharmaceutically acceptable carrier.

As used herein, "pharmaceutically acceptable carrier" includes any and all solvents, dispersion media, coatings, antibacterial and antifungal agents, isotonic and absorption delaying agents, and the like that are physiologically compatible. Preferably, the carrier is suitable for parenteral, e.g. intravenous, intramuscular, subcutaneous, intraocular or intravitreal administration (e.g., by injection or infusion). Depending on the route of administration, the composition may be coated in a material to protect the composition from the action of acids and other natural conditions that may inactivate the composition.

The pharmaceutical compositions of the invention may include one or more pharmaceutically acceptable salts. A "pharmaceutically acceptable salt" refers to a salt that retains the desired biological activity of the parent compound and does not impart any undesired toxicological effects. Examples of such salts include acid addition salts and base addition salts.

Preferred pharmaceutically acceptable carriers comprise aqueous carriers or diluents. Examples of suitable aqueous carriers that may be employed in the pharmaceutical compositions of the invention include water, buffered water and saline. Examples of other carriers include ethanol, polyols (such as glycerol, propylene glycol, polyethylene glycol, and the like), and suitable mixtures thereof, vegetable oils, such as olive oil, and injectable organic esters, such as ethyl oleate. In many cases, it will be preferable to include isotonic agents, for example, sugars, trehalose, polyalcohols such as mannitol, sorbitol, or sodium chloride in the composition.

Therapeutic compositions typically must be sterile and stable under the conditions of manufacture and storage. The composition can be formulated as a solution, microemulsion, liposome, or other ordered structure suitable to high drug concentration.

When a hydrogel is used a part of the formulation, the composition of the invention is preferably stored at 4°C before use, owing to gelation at room temperature and pressure.

Pharmaceutical compositions of the invention may comprise additional active ingredients, notably VEGF antagonists as discussed herein.

The compositions of the present invention may be administered for prophylactic and/or therapeutic treatments. In therapeutic applications, compositions are administered to a subject already suffering from a disorder or condition as described above, in an amount sufficient to cure, alleviate or partially arrest the condition or one or more of its symptoms. Such therapeutic treatment may result in a decrease in severity of disease symptoms, or an increase in frequency or duration of symptom-free periods. An amount adequate to accomplish this is defined as a "therapeutically effective amount".

In prophylactic applications, compositions are administered to a subject at risk of a disorder or condition as described above, in an amount sufficient to prevent or reduce the subsequent effects of the condition or one or more of its symptoms. An amount adequate to accomplish this is defined as a "prophylactically effective amount". Effective amounts for each purpose will depend on the severity of the disease or injury as well as the weight and general state of the subject. An example of a condition that may be treated prophylactically in the context of the invention is wet AMD (age-related macular degeneration); one eye may develop the condition before the other, with the first eye being treated once the problem is recognised and the second prophylactically.

A subject for administration of the compositions of the invention may be a human or non-human animal. The term "non-human animal" includes all vertebrates, e.g., mammals and non-mammals, such as non-human primates, sheep, dogs, cats, horses, cows, chickens, amphibians, reptiles, etc. Administration to humans is preferred.

The compositions of the present invention may be administered via one or more routes of administration using one or more of a variety of methods known in the art. As will be appreciated by the skilled artisan, the route and/or mode of administration will vary depending upon the desired results. Preferred routes of administration for compositions of the invention include intravenous, intramuscular, intradermal, intraocular, intraperitoneal, subcutaneous, spinal or other parenteral routes of administration, for example by injection or infusion. The phrase "parenteral administration" as used herein means modes of administration other than enteral and topical administration, usually by injection. Alternatively, the composition of the invention can be administered via a non-parenteral route, such as a topical, epidermal or mucosal route of administration.

In a preferred embodiment of the invention, the composition of the invention is administered topically. The composition can be administered topically to the eye in the form of an eye drop.

A suitable dosage of the composition of the invention may be determined by a skilled medical practitioner. Actual dosage levels of the active ingredients in the pharmaceutical compositions of the present invention may be varied so as to obtain an amount of the active ingredient which is effective to achieve the desired therapeutic response for a particular patient, composition, and mode of administration, without being toxic to the patient. The selected dosage level will depend upon a variety of pharmacokinetic factors including the activity of the particular compositions of the present invention employed, the route of administration, the time of administration, the rate of excretion of the particular compound being employed, the duration of the treatment, other drugs, compounds and/or materials used in combination with the particular compositions employed, the age, sex, weight, condition, general health and prior medical history of the patient being treated, and like factors well known in the medical arts.

A suitable dose may be, for example, in the range of from about $0.1\mu g/kg$ to about 100mg/kg body weight of the patient to be treated. For example, a suitable dosage may be from about $1\mu g/kg$ to about 10mg/kg body weight per day or from about 10 g/kg to about 5 mg/kg body weight per day. For intraocular administration, a suitable dosage may be from about $1\mu g$ - 1mg.

Topical formulations may contain 10 mg/mL Lucentis with 12.5 mg/mL R8. A typical eye drop volume may be 0.05 mL, therefore a typical dose may be; one drop per day: 50 $\mu g$ Lucentis with 62.5 $\mu g$ R8, two drops per day: 100 $\mu g$ Lucentis with 125 $\mu g$ R8, or four drops per day: 200 $\mu g$ Lucentis with 250 $\mu g$ R8. Dosage regimens may be, for example, every 12 hours or 24 hours. Dosage regimens may be, for example, once daily or twice daily.

Dosage regimens may be adjusted to provide the optimum desired response (*e.g.*, a therapeutic response). For example, a single dose may be administered, several divided doses may be administered over time or the dose may be proportionally reduced or increased as indicated by the exigencies of the therapeutic situation. Dosage unit form as used herein refers to physically discrete units suited as unitary dosages for the subjects to be treated; each unit contains a predetermined quantity of active compound calculated to produce the desired therapeutic effect in association with the required pharmaceutical carrier.

Administration may be in single or multiple doses. Multiple doses may be administered via the same or different routes and to the same or different locations. Alternatively, doses can be via a sustained release formulation, in which case less frequent administration is required. Dosage and frequency may vary depending on the half-life of the antagonist in the patient and the duration of treatment desired.

The composition of the invention can comprise more than one VEGF antagonist as described herein. The composition of the invention can be administered in combination with other therapies for the treatment of the conditions described herein.

Also within the scope of the present invention are kits comprising compositions of the invention. The kits may contain instructions for use. The kit may further contain one or more additional reagents, such as an additional therapeutic or prophylactic agent as discussed above.

The invention encompasses the composition of the invention as defined herein, for use in the prevention or treatment of a condition characterised by VEGF dysfunction.

The invention encompasses the composition of the invention as defined herein, for use in the prevention or treatment of a condition characterised by VEGF dysfunction, wherein the condition characterised by VEGF dysfunction is cancer, oedema or leaky vessels.

The invention encompasses the composition of the invention as defined herein, for use in the prevention or treatment of a condition characterised by VEGF dysfunction, wherein the condition characterised by VEGF dysfunction is a vasculoproliferative condition.

The invention encompasses the composition of the invention as defined herein, for use in the prevention or treatment of a condition characterised by VEGF dysfunction, wherein the vasculoproliferative condition comprises neovascularisation, vascular endothelial cell proliferation, angiogenesis, telangiectasia or microaneurysms.

The invention encompasses the composition of the invention as defined herein, for use in the prevention or treatment of a vasculoproliferative condition of the eye.

The invention encompasses the composition of the invention as defined herein, for use in the prevention or treatment of a condition characterised by VEGF dysfunction, wherein the composition is formulated for topical administration.

The invention encompasses the composition of the invention as defined herein, for use in the prevention or treatment of a condition characterised by VEGF dysfunction, wherein the composition is formulated for topical administration to the eye.

The invention encompasses the composition of the invention as defined herein, for use in the prevention or treatment of a condition characterised by VEGF dysfunction, wherein the composition is administered to the eye by eye drop.

The present invention encompasses a method of treating a tumour that exhibits vasculoproliferation as described herein, wherein the tumour is selected from brain tumour, breast tumour, kidney tumour, colorectal tumour, lung tumour, prostate tumour, head and neck tumours, stomach tumour, pancreatic tumour, skin tumour, cervical tumour, bone tumour, ovarian tumour, testicular tumour and liver tumours.

The invention encompasses the composition described herein for use in the treatment of a tumour the exhibits vasculoproliferation.

The present invention encompasses the composition described herein for use in the treatment of a tumour that exhibits vasculoproliferation as described herein, wherein the tumour is selected from brain tumour, breast tumour, kidney tumour, colorectal tumour, lung tumour, prostate tumour, head and neck tumours, stomach tumour, pancreatic tumour, skin tumour, cervical tumour, bone tumour, ovarian tumour, testicular tumour and liver tumours.

The following Examples illustrate the invention.

## EXAMPLES

We have previously established that Annexin A5 (AnxA5) functionalised liposomal nanoparticles can deliver large protein molecules including antibodies across the cornea to the back of the eye on topical application by promoting transcytosis. During scale-up of the AnxA5/Lucentis nanoparticles, we encountered two issues:

  i) the duration of the electrostatic AnxA5/nanoparticle interaction is short-lived
  ii) Lucentis interacts with nanoparticles at these concentrations causing aggregation
  which reduced effective shelf-life. Although direct covalent conjugation of AnxA5 to Lucentis loaded nanoparticles was technically feasible, it was extremely difficult and prohibitively expensive to manufacture.

Human AnxA5 contains a total of 19 arginine residues. All annexins from vertebrates contain an invariant arginine (R) in each domain. AnxA5 has a four-repeat domain structure which in turn forms higher order intermolecular structures (AnxA5 dimers/trimers) on interacting with cell membranes containing PS or PE. Although not adjacent in the primary amino acid sequence, on folding (secondary and tertiary structure), AnxA5 R residues are brought into close proximity and interact with each other via formation of intramolecular and intermolecular interactions which are key to the function of the annexin V protein.

8 of the 19 arginine residues have active membrane-binding activities. R43, R115, R199 and R274 are highly conserved arginine residues in the AnxA5 sequence, and shown to have differential effects on the PS binding ability, tertiary structure, and proteolytic susceptibility of AnxA5. R23E and R61E mutations have a significant reduction in adsorption to phospholipid membranes relative to the wildtype protein. R23 found to be major determinant for interfacial phospholipid binding and participates in an intermolecular salt bridge that is key for trimer formation on the membrane surface. R163 plays a central role in the selective binding of AnxA5 to Protein Kinase C and mutation of this residue abolishes this interaction.

AnxA5 has eight calcium binding sites involved in binding to cells and vesicles via lipid interaction.

Arginine rich peptides can be used as cell-penetrating peptides through promotion of intracellular uptake or endocytosis. This has recently been suggested to occur due through an interaction with PS and PE promoting negative membrane curvature in PS/PE containing membranes. A similar mechanism of action is proposed for AnxA5 mediated endocytosis.

Based on the above, we sought to determine whether the annexin-derived sequence RRRRRRRR (R8) (SEQ ID NO: 1) could be used as a replacement for AnxA5 initially in R8 coupled liposomes, to enhance transcytosis of encapsulated anti-VEGF across corneal epithelial barriers.

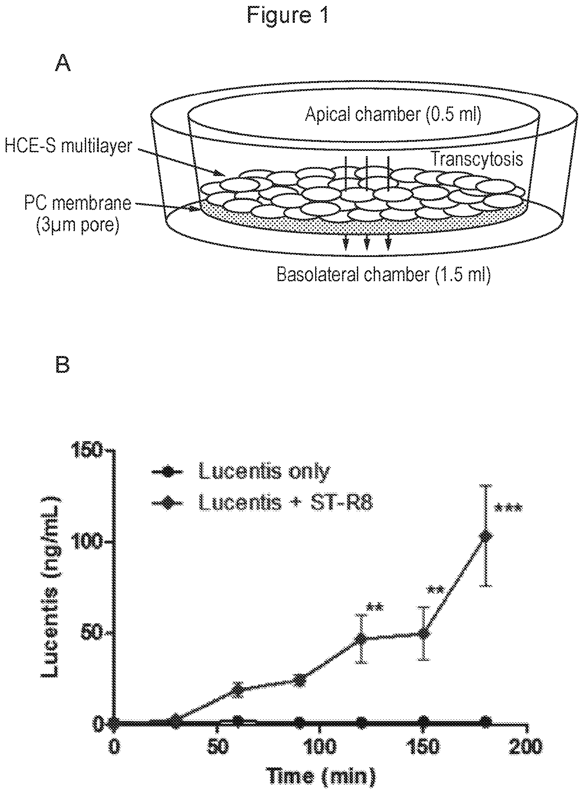

Using a previously described *in vitro* HCE-S transcytosis assay (illustration of the *in vitro* human corneal epithelial (HCE-S) barrier model used in this study shown in Figure 1), we found that the presence of ST-R8 significantly enhanced the transcytosis of anti-VEGF (Lucentis) loaded liposomes. R8 alone without liposomes was investigated as a control. Surprisingly, this formulation also showed a dramatic enhancement in transcytosis.

## Materials and Methods

### *In vitro* HCE-S assays

HCE-S cells were cultured as a monolayer in 90% DMEM supplemented with 10% heat inactivated FBS and penicillin-streptomycin (100 U/mL). Cells were cultured at 37 °C in a humidified incubator with 5% $CO_2$ and growth medium was replaced every three days. HCE-S populations were grown to 80% confluence before passage to 20% using 0.25% Trypsin-EDTA. Prior to each experiment cell populations were estimated using a haemocytometer and trypan blue exclusion assay.

### Viability assay

HCE-S monolayers for uptake assays were prepared by seeding 96 well plates with $1 \times 10^4$ cells/well and maintaining for 5 days replacing the medium every other day. After this time, medium was replaced with that containing 100 $\mu$M calcein loaded PLVs in the presence or absence of 200 nM AnxA5. Cells were incubated with PLVs at 37 °C for 3 h protected from light. Epithelial monolayers were then washed three times in ice cold PBS (with calcium and magnesium) to remove free PLVs, followed by a single wash in ice cold isosmotic citrate buffer (pH 3) to remove surface bound PLVs before applying a PBS wash containing 1% Triton X-100 to solubilise membranes. Upon completion of each washing step, calcein fluorescence was measured using a 485 nm excitation and 515 nm emission with a SAFIRE micro plate reader (TECAN) HCE-S monolayers for viability assays were prepared by seeding 96 well plates with $1 \times 10^4$ cells/well and maintaining for 24h at 37 °C in a humidified incubator with 5% $CO_2$. After this time, medium was replaced with that containing specified concentrations of anti-VEGF (Lucentis or Avastin) in the presence or absence of varying concentrations of R8 or ST-R8 (0-200 molar ratio). Cells were incubated with anti-VEGF/R8 solutions for 24 h before viability of HCE-S cells in response to was assessed using the AlamarBlue (Invitrogen) cell viability assay according to manufacturers instructions. Briefly, 10 $\mu$l AlamarBlue solution was added to each well-plate and incubated for 2.5 h after which time the intensity of AlamarBlue fluorescence (Excitation 570 nm/Emission 585 nm) was recorded from which percentage cell viability was determined using equation 1;

$$Viability\ (\%) = 100\left(\frac{(T-N)}{(H-N)}\right)$$

where T is the fluorescence intensity of the test well, H is the fluorescence intensity of a population of untreated (healthy) cells and N denotes a population of cells treated with a highly cytotoxic insult (0.1 % Triton-X100).

### Transwell barrier model establishment

HCE-S barrier models for transcytosis assays were prepared using an adaptation of a previously described method.[1] HCE-S cells ($5 \times 10^4$ cells/well) were seeded on transwell inserts (poly-carbonate, 3 $\mu$m pore size, 1.13 cm$^2$) and maintained for 7 days refreshing the medium in the apical and basal chambers every second day. After this time cells were cultured for a further 10 days at an apical air interface to encourage development of a multilayer barrier. Prior to each experiment culture medium was replaced with phenol red free DMEM and barrier integrity measured via transepithelial resistance (TER). TER values were obtained in the range 300 - 600 $\Omega$ cm$^2$ and only epithelial barriers with TERs > 300 $\Omega$.cm$^2$, the accepted threshold for tight epithelial barriers, were used for transcytosis experiments.

### Anti-VEGF transcytosis experiments

Permeation studies were conducted to determine the rate of anti-VEGF transcytosis in the presence/absence of varying concentrations of R8. Experiments were initiated by adding 1.5 ml serum- and phenol red-free DMEM to the basal chamber and 0.5 ml to the apical chamber containing 0.1 mg/mL Avastin or Lucentis in the presence of varying molar ratios of R8. After 3 h, 100 $\mu$l of medium was withdrawn from the basal chamber and the concentration of anti-VEGF in the basal chamber was determined using established and commercially available sandwich ELISA techniques (KPL Protein Detector HRP Microwell Kit, Anti-Human Cat# 54-62-10 with Jackson Immuno Research [309-005-082] AffiniPure Rabbit Anti-Human IgG capture antibody).[1] The presence of R8 was found to not significantly impact anti-VEGF detection using this ELISA technique. The permeability coefficient ($P_{app}$) of each anti-VEGF was determined as previously described[1] using equation 1;

$$P_{app} = \left( \frac{(C_R / C_D)}{t_{min}} \right) * \left( \frac{V_R}{(A * 60)} \right)$$

where $C_R$ is the final concentration of anti-VEGF recovered from the ELISA assay, $C_D$ is the initial concentration of anti-VEGFs added to the assay, $t_{min}$ is the transcytosis time (180 min), $V_R$ is the volume of the receiving chamber (1.5 ml) and A represents the filter area (1.12 cm$^2$).

**Stability of R8 containing formulations**

Lucentis (0.1 mg/mL) was added to each well of a 96 well plate in the presence of varying concentrations of R8 in the same buffer (10 mM Histidine HCL, 10% (w/v) Trehalose and 0.1% (v/v) polysorbate 80) and additional trehalose was added (up to a final concentration of 600 mg/mL). Samples were incubated at 25°C and aggregation monitored by recording absorbance at 600 nm over time using a TECAN Safire plate-reader.

Anti-VEGF activity was recorded using a VEGF binding ELISA. Briefly, a Nunc Maxisorp (cat 442404) high affinity, protein binding ELISA plate was coated in 0.05 mL of 0.25 $\mu$g/mL human **VEGF$_{165}$** Peprotech [Cat #100-20] dissolved in PBS buffer overnight at 4°C. Plates were washed (three times, five minutes each) with Tris-buffered saline containing 0.005% Tween-20 before blocking for 1.5h with Protein free (TBS), Thermo scientific (Cat #37570). Plate was washed before samples and standards of known anti-VEGF concentrations were added and the plate incubated for a further 2h. After this time the plate was washed before 0.05 mL/well of 0.1 mg/mL of detection antibody was applied (Affinity purified Goat anti-Human IgG Peroxidase labelled (KPL, 04-10-06)) for a further 1h. Finally, the plate was washed before developing with BM Blue POD substrate, soluble (Sigma, 000000011484281001) for 10 minutes before stopping the reaction with 1M Hydrochloric acid and recording absorbance at 450 nm against a reference wavelength of 690nm. Results are presented as the ratio of $IC_{50}$ of each formulation over the $IC_{50}$ of commercially available anti-VEGF formulations.

*In vivo* **Rabbit studies**

All animal experiments were performed in compliance with the ARVO Statement for the Use of Animals in Ophthalmic and Vision Research. New Zealand rabbits received unilateral topical instillations of 0.03 mL anti-VEGF in the presence of R8 (varying molar concentrations) either as a liquid or hydrogel formulations without anaesthesia. At the specified time after application animals were euthanized before enucleation, washing (PBS) and snap-freezing and dissection of ocular tissues. Samples were weighed and cryohomogenised before the concentration of anti-VEGF per gram of eye tissue determined using established sandwich ELISA protocols.

Corneal toxicity was assessed *in vivo* in rabbit eyes, where evidence of redness, swelling, discharge, ulceration, haemorrhaging or scarring in the treated eye was assessed. Epithelial toxicity was investigated using fluorescein staining, using standard protocols.

Reference

[1] B. Davis, E. Normando, L. Guo, P. O'Shea, S. Moss, S. Somavarapu, M. Cordeiro, Small 2014, 10, 1575.

**Example 1- R8/anti-VEGF molar ratios and transcytosis**

Initial formulations concerned replacing AnxA5 with R8 in liposomal and micelle formulations, but we found that electrostatic interactions between R8 and protein drugs also had a beneficial effect for drug delivery. A schematic of the *in vitro* HCE-S assay used to measure transport across corneal barriers *in vitro* is shown in **Figure 1A**. **Figure 1 B** indicates that presence of R8 (Steryl-R8) enhances the transport of Lucentis across corneal barriers *in vitro*. Lucentis (+/- Steryl-R8) were applied to the Apical chamber and the concentration crossing the barrier was determined from the basolateral chamber at different timepoints using a Lucentis sandwich ELISA. Intact HCE-S barrier was confirmed by measuring transepithelial resistance (> 600 $\Omega$.cm$^2$) before each assay.

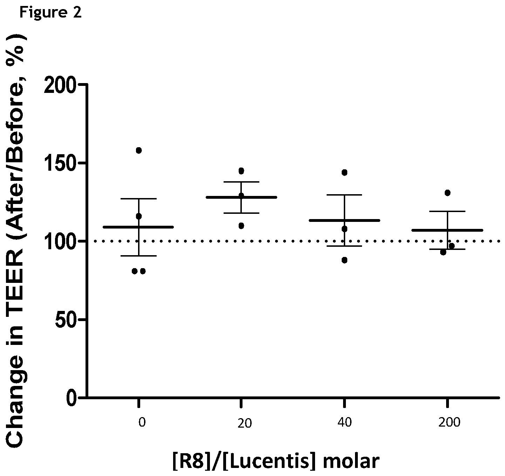

R8 enhanced transcytosis across cornea via a transcellular mechanism without affecting cell viability or disruption of corneal barrier integrity (**Figure 2**). Addition of R8 and Lucentis to HCE-S transwells for 3h induced no significant change in transepithelial resistance suggestive of a transcellular delivery mechanism. This is contrary to what has been reported with poly-arginine (36 kDa) polypeptides for which the mechanism has been suggested to be reversible disruption of tight junctions (TJs) (Nemoto et al. Biol Pharm Bull. 2007 Sep;30(9): 1768-72.)

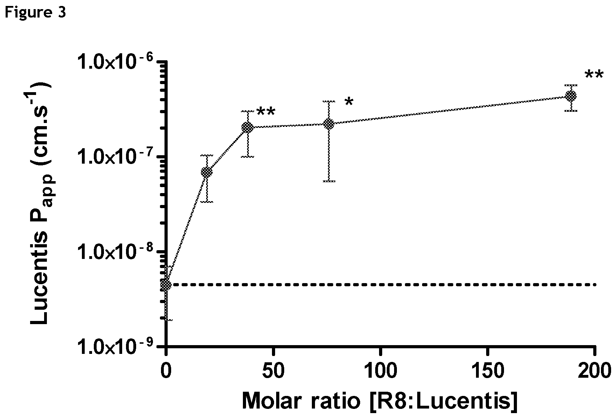

R8 enhanced delivery across corneal barriers *in vitro* in a concentration dependent manner. A surprising finding in our *in vitro* studies was that there is evidence that R8 promoted delivery across corneal cells. Another method to monitor the extent of delivery across corneal barrier models is to calculate the drug permeability coefficient ($P_{app}$) as described in Davis et al 2014. **Figure 3** shows that R8 enhanced the delivery of the anti-VEGF Lucentis across the HCE-S barrier in a concentration dependent manner. Molar ratios of R8:Lucentis of 40:1 or more significantly enhanced corneal transcytosis *in vitro*. **Figure 4** shows that R8 enhances the delivery of the both VEGF antagonists Avastin and Lucentis across the HCE-S barrier. In addition, Stearyl-R8 enhanced the delivery of the anti-VEGF Lucentis across the HCE-S barrier in a concentration dependent manner.

## Example 2- Toxicity and stability testing

Using the alamarBlue cell viability assay R8 was found to be non-toxic to *in vitro* human corneal cell cultures (HCE-S) after 24h exposure. (**Figure 5**). R8 was found to be well tolerated in the absence or presence of Lucentis after 24h exposure. R8 caused no significant reduction in cell viability at the highest concentrations assessed (200/1 molar ratio).

The presence of R8 did not negatively affect the VEGF binding activity of Lucentis. (**Figure 6**). The presence of R8 did not impact the Lucentis sandwich ELISA used in rabbit studies. Using a hVEGF-165 ELISA to assess Lucentis activity, the addition of R8 in a molar ratio of 200/1 of R8/Lucentis caused no significant impediment in Lucentis binding to hVEGF. In fact the data suggested that the presence of these peptides may mildly increase the affinity of Lucentis for hVEGF. Similar results were achieved using Avastin (**Figure 7**).

The stability of the R8:Lucentis formulations were investigated by measuring aggregation over time using 600nm absorbance at 25°C (**Figure 8**). Ratios of 50 to 100:1 R/L were the most stable. However, aggregation was delayed by increasing the Trehalose concentration in the formulation. The results suggest a ratio of 50:1 would be suitable the formulation of the invention. Similar results were seen by reducing Lucentis concentration while maintaining the R/L ratio, or by increasing the Trehalose concentration further, to 600mg/mL (**Figure 9**). R8/Lucentis combinations at ratios of 40:1 and 80:1 were found to be stable for 3 months at 4°C (**Figure 10A**). An R8/Lucentis combination at a ratio of 50:1 was also found to be stable for 3 months (12 weeks) at 4°C (**Figure 10B**).

In addition, the presence of R8 did not interfere with the anti-VEGF detection ELISA (**Figure 11**).

## Example 3- *In vivo* studies

A control *in vivo* experiment to determine the proportion of free Lucentis reaching the retina is shown in **Figure 12**. The proportion of dosed free Lucentis reaching the retina by the topical route was ~30%. Furthermore, R8:Lucentis at a ratio of 20/1 did not enhance delivery of Lucentis to the retina after 2h. A 100 mg/mL Lucentis with 50 mg/mL R8 combination provided no significant improvement in Lucentis delivery to the retina compared to Lucentis only control (n=3) (16+/- 6ng/g versus 18+/- 8ng/g). (**Figure 13**). This suggests that the R8:Lucentis ratio is important. This ratio of R peptide to anti-VEGF is similar to that reported in the de Logan (2017) reference, suggesting issues with the data presented in that reference. The above data is supported by the *in vitro* transwell data that suggested that 40:1 ratio is required at minimum.

However, a ratio of R8 to Lucentis of 200/1 enhanced delivery to the retina after 2h (**Figure 14**), see retinal Lucentis level in bottom graph, in comparison with equivalent graph in Figure 12. **Figure 15** summaries the results of the *in vivo* assessment of Lucentis concentrations in the retina after topical application of R/L formulations at 2 h.

## Example 4- Hydrogel formulation R8:Lucentis

To increase corneal contact time, R8:Lucentis formulations were made with hydrogels to enhance formulation viscosity and their properties were investigated. Figure 16 shows that by increasing the concentration of a hydrogel in the formulation, a thermosensitive gel was formed with the desired properties. In the experiment shown in **Figure 16**, Lutrol F127 was used as the hydrogel. Solutions were prepared by mixing the desired quantities of Lutrol F127 with 20mM HEPES buffer, pH 7.4. Between 20% and 25% Lutrol was the optimal concentration range tested. The R8: Lucentis formulation was stable at 4°C for seven days in the presence of increasing concentrations of Trehalose (**Figure 17A**). In addition, reducing the Lucentis concentration whilst keeping the ratio of R8:Lucentis the same, aided stability at higher ratios. Furthermore the 20% Lutrol hydrogel improved the stability of Lucentis formulations. The presence of Lutrol helped prevent aggregation of R8:Lucentis formulations even at 200:1 ratio. **Figure 17B** shows that a formulation of R8:Lucentis (50:1) and hydrogel (20% Lutrol) was stable for 12 weeks at 4°C. **Figure 17C** shows that a formulation of R8:Lucentis (50:1) and hydrogel (20% Lutrol) remained stable for 12 hours at 4°C and 25°C. **Figure 17D** shows that a formulation of R8:Lucentis (50:1), hydrogel and the mucoadhesive polymer 5K chitosan (01% w/v) showed good stability for 4 weeks as determined by $EC_{50}$ ratio (U8:Lucentis / Standard) of less than 2.

**Figure 18A** shows that a formulation of 20% Lutrol gel, 10mm HisCl, 10% trehalose, 0.01% polysorbate 20, pH 5.5 took approximately 1 hr to release Lucentis in a release assay. **Figure 18B** summaries the stability results for the R8/Lucentis liquid and hydrogel formulations. R8/Lucentis ratios of up to/ including 100:1 in liquid formulation were stable in a concentration of 100 or 600 mg/ml Trehalose. In a 20% Lutrol hydrogel formulation R8/Lucentis ratios of up to/ including 200:1 were stable in concentrations of 100-600 mg/ml Trehalose.

Incorporation of Lucentis/R8 into a hydrogel dramatically increased the concentration of anti-VEGF delivered to posterior ocular tissues of the rabbit, due to enhanced corneal contact time (**Figure 19**). **Figure 19** shows that R8/Lucentis 200:1 ratio gel delivered significant concentrations of Lucentis to the retina at 2h **(A)** and 12h **(B)** post hydrogel administration. At both timepoints the Lucentis concentration exceeded the target concentration in the retina of at least 200ng/g retinal tissue. The data is summarised in **Figure 19C. Figure 20** shows that R8/Lucentis 50:1 ratio gel delivered significant concentrations of Lucentis to the retina at 2h **(A)**, 12h **(B)** and 24h **(C)** post hydrogel administration. At 2h and 12h timepoints the Lucentis concentration exceeded the target concentration in the retina of at least 200ng/g retinal tissue. At 24h post hydrogel administration the Lucentis concentration had dropped below the retinal target level of 200ng/g retinal tissue. The data is summarised in **Figure 20D**.

**Figure 21A** summarises the *in vivo* assessment of Lucentis concentrations in the retina after topical application of R/L formulations at 2h, 12h, 24h and 72h, with the addition of a hydrogel and chitosan as indicated. A R8/Lucentis 80:1 ratio hydrogel formulation with 0.1% w/v chitosan seemed to give the best retinal Lucentis profile.

**Figure 21B** shows that the preferred U8:Lucentis formulation (80:1 R8 to Lucentis ratio, hydrogel, trehalose and chitosan) is not associated with redness or corneal damage *in vivo* in rabbit eyes, as seen by fluorescein staining.

**Figure 22** summaries the experiments conducted, and ranges of R8/Lucentis ratios tested, to come to the conclusion that a ratio range of 50-80:1 R8 to Lucentis is the optimal ratio range.

**Example 5- Investigation into the mechanism of R8/Lucentis interaction**

The interaction between R8 and Lucentis was investigated further. One hypothesis considered was that as R8 is highly charged, it may electrostatically associate with the protein surface giving the particle a net positive charge, so enhancing drug delivery across ocular membranes. Another hypothesis considered was the bystander effect. R8 could destabilize cell membranes through the interaction with lipid phosphate groups. R8 mediated disruption of the ocular tear film and corneal epithelial plasma membrane could thus enhance the passive diffusion of drugs into ocular tissues.

The first experiment conducted was Size Exclusion Chromatography (SEC), **Figure 23**. There was no co-elution of of Lucentis and FITC-R8, so no binding. The second experiment conducted was Quartz Crystal Microbalance (QCM), **Figure 24**. This method measures a mass variation per unit area by measuring the change in frequency of a quartz crystal resonator. The resonance is disturbed by the addition or removal of a small mass due to oxide growth/decay or film deposition at the surface of the acoustic resonator.

**Sauerbrey Equation**

$$\Delta f = -C_f \, . \, \Delta m$$

Where $\Delta f$ — the observed frequency change in Hz.

$\Delta m$ — the change in mass per unit area, in $g/cm^2$, and
$C_f$ — the sensitivity factor for the crystal used (i.e. 56.6 Hz $\mu g^{-1}$ $cm^2$ for a 5MHz AT-cut quartz crystal at room temperature)

Attana QCM system; approx.. 4.4 Hz/( $ng*cm^2$) which further reduces to 0.7 Hz/ng (1.5 $mm^2$)

$$75*0.7 = 52.5 \text{ ng (11.3 pmol of Lucentis bound)}$$

Hypothetical calculations: 1mm depth= 1.5 mm$^3$ (1.5 $\mu$l)
20 $\mu$M R8 in 1.5 mm$^3$ = 30pmol R8 (R8:Lucentis = 2.6)
200 $\mu$M R8 in 1.5 mm$^3$ = 300pmol R8 (R8:Lucentis = 26)

1 $\mu$L/s flow rate, 20 $\mu$M (0.26-2.6): 1 ratio, 200 $\mu$M (2.6-26) ratio R8: Lucentis, control: 4Hx difference = 5.7 ng VEGF 165 (6.25 $\mu$g added).
Lucentis was immobilised on a chip surface and tested, no binding to R8 was detected.
The third experiment conducted was Surface Plasmon Resonance (SPR), **Figure 25**. Lucentis was immobilised on a CM5 chip surface RU = 3700. Increasing concentrations of R8 we tested either in buffer of 10mM HisCl pH 5.5, 10% Trehalose or 10mM HEPES, pH7.4, 150 mM NaCl, 3 mM EDTA and 0.05% v/v surfactant P20. Some binding of R8 to Lucentis was detected.
The fourth experiment conducted was Isothermal titration calorimetry (ITC) (**Figure 26**). This is a technique used to determine the thermodynamic parameters of interactions in solution. It is most often used to study the binding of small molecules (such as medicinal compounds) to larger macromolecules (proteins, DNA etc.) ITC is a quantitative technique that can determine the biding affinity, enthalpy changes and binding stoichiometry of the interaction between two or more molecules on solution. From these initial measurements, Gibbs energy changes and entropy changes can be determined using the relationship; dG= - RTInKa = dH- TdS. No binding between R8 and Lucentis was detected at ratios up to 180 R8: Lucentis.
The fifth experiment conducted was whether R8 affected the transport of dextrans across a membrane, **Figure 27**. Dextrans are complex branched polysaccharides made of many glucose molecules. They can be composed of chains of varying lengths. In a dextran transcytosis assay, used to check if R8 carried dextrans of various sizes across the membrane, R8 made no difference to the transport of the different sizes of dextran.
The results of the experiments are summarised in **Figure 28**. Due to the lack of evidence for binding to Lucentis, it seems that R8 may be working via the bystander effect. If so, then the action of R8 would aid in the transport of a variety of macromolecules and drugs across membranes.

**Example 6 - Exemplified product specification and method of manufacture**

**HICF product preparation:**

**Day 1**

1. **Buffer A preparation:** 20% Lutrol F127, 0.1% w/v 5k chitosan, 20 mg/mL R8 peptide:
a. To 40 mL of pre-chilled (4°C) distilled water add the following with mixing (4 °C roller for 1-3h);

| Constituent | Molecular weight (Da) | Amount required |
|---|---|---|
| Lutrol F127 | 15000 | 10 g |
| 5k Chitosan | 5000 | 50 mg |
| R8 peptide | 1267.51 | 500 mg |

b. Make up to 50 mL with additional chilled distilled water
c. Mix 50 mL of Buffer A with 50 mL of Lucentis (as provided) 4 °C roller for 1h
d. 0.2 $\mu$m filter the resulting solution and store at 4°C until required (ideally use immediately)
e. Prepare 2 mL aliquots of this material into 6 mL glass vials[2]

**2. Freeze-drying (conditions will require optimisation - seeks to achieve a x2 fold concentration of product)**

a. Freezing: -60 °C, 2h
b. Primary Drying: -34 °C, 100 mTorr, 18h
c. Secondary Drying: 20 °C, 100 mTorr, 2h
d. Store cakes at 4 °C until required, protecting from light

Day 2

### 3. Rehydration (performed in 4 °C cold room)

a. To a freeze-dried cake (prepared in 1-2 above) add 1 mL (half-pre-freeze drying volume to give a two-fold concentrating effect) of pre-chilled sterile distilled water. Rehydrate with rotation (tube roller 2 h at 4 °C and 30 rpm or until cake is fully dissolved).
b. 0.2 $\mu$m filter the resulting solution and aliquot into vials.
c. **Approximate Final composition (may be additional dilution effects due to high solute concentration)**

10 mg/mL Lucentis
20 mg/mL R8
20% Lutrol F127
10 mM histidine HCl
10% w/v $\alpha,\alpha$-trehalose dihydrate
0.01% w/v polysorbate 20
*0.1% w/v 5k Chitosan*
pH 5.5

NOTES

1. Suggested filter type: Millipore® Stericup™ filter units PVDF membrane (Durapore), very low protein binding, pore size 0.22 $\mu$m, funnel capacity 150 mL CAT NO: #SCGVU01RE, available from http://www.merckmillipore.com/GB/en/product/Stericup-GV-Sterile-Vacuum-Filtration-System,MM_NF-SCGVU01RE
2. Alternative is to freeze-dry in bulk and rehydrate in bulk before sterile filtering and aliquotting the resulting solution

### Example 7 - Appendix SOP for Lucentis activity ELISA

**Day 1.**

### Plate coating

1. Defrost one aliquot of hVEGF$_{165}$ per plate and add 5.40 mL of coating buffer to the Eppendorf tube (0.25 $\mu$g/mL)
2. Coat each well of a 96 well plate with 50 $\mu$L/well of the hVEGF$_{165}$ solution
3. Tap the plate to ensure good coverage before covering and incubating at 4 °C overnight

**Day 2.**

### Blocking (2h, prepare Lucentis dilutions in the gap)

1. Wash plate three times with 300 $\mu$L/well wash buffer. Each wash should be left on the plate for 5 minutes
2. Block non-specific binding sites with 300 $\mu$L/well of blocking buffer. Incubate the sealed plate for 1.5h with shaking.

### Lucentis dilutions (2 h, based on scFv dilution protocol)

| Dilution buffer: | To 50 mL of PBS add 0.5g BSA and 100 $\mu$L of Tween 20. Mix at room temperature to dissolve and filter (0.2 $\mu$m) before use. |
|---|---|

1. Wash plate three times with 300 μL/well wash buffer. Each wash should be left on the plate for 5 minutes

2. For the standard curve, prepare the following dilutions in dilution buffer or matrix;

a. Dilute 0.05 mL Lucentis stock solution (10 mg/mL) into 4.95 mL of dilution buffer **[Solution A,** 100 μg/mL]

b. Dilute 0.05 mL of **[Solution A]** into 4.95 mL of dilution buffer **[Solution B,** 1000 ng/mL**]**

c. Dilute 0.05 mL of [**Solution A**] into 4.95 mL of dilution buffer with 1μg/mL of R8 (5 μL of 1 mg/mL stock solution) [**Solution B-R,** 1000 ng/mL]

d. Dilute 0.15 mL of [**Solution B**] into 1.35 mL of Dilution buffer **[Solution 1,** 100 ng/mL**] (make x4)**

e. Dilute 0.15 mL of [**Solution B-R**] into 1.35 mL of Dilution buffer **[Solution 1-R,** 100 ng/mL**] (make x4)**

f. **From Solutions 1 and 1-R prepare the following dilution range (x4 each);**

| Solution | Lucentis (ng/mL) | Volume of stock (μL) | Stock | Volume of dilutant (μL) |
|----------|------------------|----------------------|-------|-------------------------|
| 1 | 100 | 750 | 1 | 0 |
| 2 | 60 | 420 | 1 | 280 |
| 3 | 40 | 400 | 1 | 200 |
| 4 | 20 | 250 | 3 | 250 |
| 5 | 10 | 250 | 4 | 250 |
| 6 | 5 | 250 | 5 | 250 |
| 7 | 1 | 100 | 6 | 400 |
| 8 | 0.2 | 100 | 7 | 400 |

g. Add 50 μL of each to the plate and incubate for 2h with gentle shaking.

| | 1 2 3 | 4 5 6 | 7 8 9 | 10 11 12 |
|---|-------|-------|-------|----------|
| **A** | 100 ng/mL | 100 ng/mL | 100 ng/mL | 100 ng/mL |
| **B** | 60 ng/mL | 60 ng/mL | 60 ng/mL | 60 ng/mL |
| **C** | 40 ng/mL | 40 ng/mL | 40 ng/mL | 40 ng/mL |
| **D** | 20 ng/mL | 20 ng/mL | 20 ng/mL | 20 ng/mL |
| **E** | 10 ng/mL | 10 ng/mL | 10 ng/mL | 10 ng/mL |
| **F** | 5 ng/mL | 5 ng/mL | 5 ng/mL | 5 ng/mL |
| **G** | 1 ng/mL | 1 ng/mL | 1 ng/mL | 1 ng/mL |
| **H** | 0.2 ng/mL | 0.2 ng/mL | 0.2 ng/mL | 0.2 ng/mL |

**Antibody coating (1.5 h)**

3. Wash plate three times with 300 μL/well wash buffer. Each wash should be left on the plate for 5 minutes

| Anti-human HRP IgG: | **From lyophilised stock** |
|---------------------|-----------------------------|
| | Rehydrate peroxidase-labelled anti-human IgG antibody with 1 mL of 50% glycerol solution to yield a concentration of 0.1 mg/mL. Solution is stable a 4 °C for 1 year. |
| | **Working solution (From avastin assay)** |
| | Add 3 μL of antibody stock solution to 10 mL of dilution buffer, mix well before use. |

4. Add 100 μL of anti-human HRP-IgG to each well, react for 1h at room temperature

**Plate Development (1 h)**

5. Wash plate three times with 300 μL/well wash buffer. Each wash should be left on the plate for 5 minutes

6. Turn off the lights

7. Add 50 μL/well POD substrate

8. Incubate for 15 minutes

9. Stop the reaction by adding 50 μL/well of 1M HCl

10. Measure absorbance at 450 nm against a reference wavelength of 690nm Lucentis activity note: http://www.ema.europa.eu/docs/en_GB/document_library/EPAR__Scientific_Discussion/human/000715/WC500043550.pdf

"Ranibizumab dose-dependently inhibited VEGF-induced proliferation in human umbilical vein endothelial cell (HUVEC), which are cells that express the VEGF-receptors, with IC50 values below 1 nM. This is 10-20-fold over clinical Cmax. Roughly approximated IC10 levels were somewhat higher than the clinical Cmax values (1.7 ng/mL predicted at steady state, maximal individual level 2.4 ng/mL) and consequently, no significant systemic VEGF-inhibiting activity is expected in humans. A total inhibition of proliferation was observed at ranibizumab concentrations $\geq$ 1.3 nM. Ranibizumab inhibited rhVEGF165-induced tissue factor up-regulation in a dose-dependent manner, with an IC50 of 0.31 nM."

## REFERENCES

F. de Cogan, L. J. Hill, A. Lynch, P. J. Morgan-Warren, J. Lechner, M. R. Berwick, A. F. A. Peacock, M. Chen, R. A. H. Scott, H. Xu, A. Logan, Investig. Opthalmology Vis. Sci. 2017, 58, 2578.

B. Davis, E. Normando, L. Guo, P. O'Shea, S. Moss, S. Somavarapu, M. Cordeiro, Small 2014, 10, 1575.

F. Oling, W. Bergsma-Schutter, A. Brisson, J. Struct. Biol. 2001, 133, 55.

B. Campos, S. Wang, G. S. Retzinger, M. A. Kaetzel, B. A. Seaton, N. J. Karin, J. D. Johnson, J. R. Dedman, Arch. Med. Res. n.d., 30, 360.

Y. Mo, B. Campos, T. R. Mealy, L. Commodore, J. F. Head, J. R. Dedman, B. A. Seaton, J. Biol. Chem. 2003, 278, 2437.

V. Kheifets, R. Bright, K. Inagaki, D. Schechtman, D. Mochly-Rosen, J. Biol. Chem. 2006, 281, 23218.

M. Jin, C. Smith, H.-Y. Hsieh, D. F. Gibson, J. F. Tait, 2004, DOI 10.1074/jbc.M405846200.

M. Vazdar, E. Wernersson, M. Khabiri, L. Cwiklik, P. Jurkiewicz, M. Hof, E. Mann, S. Kolusheva, R. Jelinek, P. Jungwirth, J. Phys. Chem. B 2013, 117, 11530.

K. Melikov, A. Hara, K. Yamoah, E. Zaitseva, E. Zaitsev, L. V Chernomordik, Biochem. J 2015, 471, 221.

Z. Wu, Q. Cui, A. Yethiraj, J. Phys. Chem. B 2013, 117, 12145.

H. Kenis, H. van Genderen, N. M. Deckers, P. a G. Lux, L. Hofstra, J. Narula, C. P. M. Reutelingsperger, Exp. Cell Res. 2006, 312, 719.

H. Kenis, H. van Genderen, A. Bennaghmouch, H. a Rinia, P. Frederik, J. Narula, L. Hofstra, C. P. M. Reutelingsperger, J. Biol. Chem. 2004, 279, 52623.

J. H. Trueblood, R. M. Rossomondo, L. A. Wilson, W. H. Carlton, Arch. Ophthalmol. (Chicago, Ill. 1960) 1975, 93, 127.

Y. Saishin, Y. Saishin, K. Takahashi, R. L. e Silva, D. Hylton, J. S. Rudge, S. J. Wiegand, P. A. Campochiaro, J. Cell. Physiol. 2003, 195, 241.

L. Yu, X. Wu, Z. Cheng, C. V. Lee, J. LeCouter, C. Campa, G. Fuh, H. Lowman, N. Ferrara, IOVS 2008, 49, 522.

H. Gerber, X. Wu, L. Yu, C. Wiesmann, X. Liang, C. Lee, G. Fuh, C. Olsson, L. Damico, D. Xie, Y. Meng, J. Gutierrez, R. Corpuz, B. Li, L. Hall, L. Rangell, R. Ferrando, H. Lowman, F. Peale, N. Ferrara, PNAS 2007, 104, 3478.

W.-C. Liang, X. Wu, F. V. Peale, C. V. Lee, Y. G. Meng, J. Gutierrez, L. Fu, A. K. Malik, H.-P. Gerber, N. Ferrara, G. Fuh, J. Biol. Chem. 2006, 281, 951.

C. Fischak, et al. J. Ophthalmology 2017, Article ID 5192924, 6 pages D. Schmidl, et al., J. Ocular Pharmacology and Therapeutics, 2017, 33, 375-382.

**Claims**

1. A composition comprising the peptide RRRRRRRR (SEQ ID NO: 1) and Ranibizumab, wherein the molar ratio of peptide to Ranibizumab is at least 40:1.

2. The composition of claim 1, wherein the molar ratio of peptide to Ranibizumab is at least 50:1, optionally 50:1 to 80:1.

3. The composition of claim 1 or 2, wherein the composition also comprises Trehalose.

4. The composition of claim 3, wherein Trehalose is present at a concentration of 100mg/ml or more, 200mg/ml or more, 300mg/ml or more, 400mg/ml or more, 500 mg/ml or more, or 600 mg/ml or more.

5. The composition of any of the preceding claims, wherein the composition also comprises a hydrogel.

6. The composition of claim 5, wherein the hydrogel concentration is between 15% and 25% w/v, optionally between 20% to 25% w/v.

7. The composition of any of the preceding claims, wherein the composition also comprises chitosan.

8. The composition of claim 7, wherein the chitosan concentration is between 0.05% and 0.25% w/v, optionally wherein the chitosan concentration is 0.1% w/v.

9. The composition according to any of claims 1 to 8, for use in the prevention or treatment of a condition **characterised by** VEGF dysfunction.

10. The composition for use according to claim 9, wherein the condition **characterised by** VEGF dysfunction is cancer, oedema or leaky vessels.

11. The composition for use according to claim 9, wherein the condition **characterised by** VEGF dysfunction is a vasculoproliferative condition, optionally wherein the vasculoproliferative condition comprises neovascularisation, vascular endothelial cell proliferation, angiogenesis, telangiectasia or microaneurysms.

12. The composition according to any of claims 1 to 8, for use in the prevention or treatment of a vasculoproliferative condition of the eye, optionally wherein the vasculoproliferative condition of the eye is selected from age-related macular degeneration (AMD), diabetic retinopathy, retinal vein occlusion, retinopathy of prematurity, macular telangiectasia, or choroidal neovascularisation, diabetic vasculopathy, diseases **characterised by** retinal angiogenesis and sickle cell disease.

13. The composition for use according to any one of claims 9 to 12, wherein the composition is formulated for topical administration.

14. The composition for use according to claim 13, wherein the composition is formulated for topical administration to the eye, optionally for administration to the eye by eye drop.

15. A kit comprising the composition as defined in any of claims 1 to 8.

**Patentansprüche**

1. Zusammensetzung, die das Peptid RRRRRRRR (SEQ ID NO: 1) und Ranibizumab umfasst, wobei das Molverhältnis von Peptid zu Ranibizumab mindestens 40:1 beträgt.

2. Zusammensetzung nach Anspruch 1, wobei das Molverhältnis von Peptid zu Ranibizumab mindestens 50:1, optional 50:1 bis 80:1 beträgt.

3. Zusammensetzung nach Anspruch 1 oder 2, wobei die Zusammensetzung auch Trehalose umfasst.

4. Zusammensetzung nach Anspruch 3, wobei Trehalose in einer Konzentration von 10 mg/ml oder mehr, 200 mg/ml oder mehr, 300 mg/ml oder mehr, 400 mg/ml oder mehr, 500 mg/ml oder mehr oder 600 mg/ml oder mehr vorhanden ist.

5. Zusammensetzung nach einem der vorhergehenden Ansprüche, wobei die Zusammensetzung auch ein Hydrogel umfasst.

6. Zusammensetzung nach Anspruch 5, wobei die Hydrogelkonzentration zwischen 15 % und 25 % Gew./Vol., optional zwischen 20 % und 25 % Gew./Vol. beträgt.

7. Zusammensetzung nach einem der vorhergehenden Ansprüche, wobei die Zusammensetzung auch Chitosan umfasst.

8. Zusammensetzung nach Anspruch 7, wobei die Chitosankonzentration zwischen 0,05 % und 0,25 % Gew./Vol. beträgt, wobei die Chitosankonzentration optional 0,1 % Gew./Vol. beträgt.

9. Zusammensetzung nach einem der Ansprüche 1 bis 8 zur Verwendung bei der Vorbeugung oder Behandlung eines durch VEGF-Dysfunktion gekennzeichneten Zustands.

10. Zusammensetzung zur Verwendung nach Anspruch 9, wobei der durch VEGF-Dysfunktion gekennzeichnete Zustand Krebs, Ödeme oder undichte Gefäße ist.

11. Zusammensetzung zur Verwendung nach Anspruch 9, wobei der durch VEGF-Dysfunktion gekennzeichnete Zustand ein vaskuloproliferativer Zustand ist, optional wobei der vaskuloproliferative Zustand Neovaskularisation, vaskuläre Endothelzellproliferation, Angiogenese, Teleangiektasien oder Mikroaneurysmen umfasst.

12. Zusammensetzung nach einem der Ansprüche 1 bis 8, zur Verwendung bei der Vorbeugung oder Behandlung eines vaskuloproliferativen Zustands des Auges, optional wobei der vaskuloproliferative Zustand des Auges aus Folgendem ausgewählt ist:
altersbedingte Makuladegeneration (Age-related Macular Degeneration, AMD), diabetische Retinopathie, Netzhautvenenverschluss, Frühgeborenenretinopathie, Makula-Teleangiektasien oder choroidale Neovaskularisation, diabetische Vaskulopathie, Erkrankungen, die durch retinale Angiogenese und Sichelzellanämie gekennzeichnet sind.

13. Zusammensetzung zur Verwendung nach einem der Ansprüche 9 bis 12, wobei die Zusammensetzung zur topischen Verabreichung formuliert ist.

14. Zusammensetzung zur Verwendung nach Anspruch 13, wobei die Zusammensetzung zur topischen Verabreichung an das Auge, optional zur Verabreichung an das Auge durch Augentropfen formuliert ist.

**15.** Kit, das die Zusammensetzung nach einem der Ansprüche 1 bis 8 umfasst.

**Revendications**

**1.** Composition comprenant le peptide RRRRRRRR (SEQ ID NO : 1) et du ranibizumab, dans laquelle le rapport molaire du peptide par rapport au ranibizumab est d'au moins 40 : 1.

**2.** Composition selon la revendication 1, dans laquelle le rapport molaire du peptide par rapport au ranibizumab est d'au moins 50 : 1, éventuellement de 50 : 1 à 80 : 1.

**3.** Composition selon la revendication 1 ou 2, dans laquelle la composition comprend également du tréhalose.

**4.** Composition selon la revendication 3, dans laquelle le tréhalose est présent à une concentration de 100 mg/ml ou plus, 200 mg/ml ou plus, 300 mg/ml ou plus, 400 mg/ml ou plus, 500 mg/ml ou plus, ou 600 mg /ml ou plus.

**5.** Composition selon l'une quelconque des revendications précédentes, dans laquelle la composition comprend également un hydrogel.

**6.** Composition selon la revendication 5, dans laquelle la concentration en hydrogel est comprise entre 15 % et 25 % p/v, éventuellement entre 20 % et 25 % p/v.

**7.** Composition selon l'une quelconque des revendications précédentes, dans laquelle la composition comprend également du chitosane.

**8.** Composition selon la revendication 7, dans laquelle la concentration en chitosane est comprise entre 0,05 % et 0,25 % p/v, éventuellement dans laquelle la concentration en chitosane est de 0,1 % p/v.

**9.** Composition selon l'une quelconque des revendications 1 à 8, à utiliser dans la prévention ou le traitement d'une affection **caractérisée par** un dysfonctionnement du VEGF.

**10.** Composition à utiliser selon la revendication 9, dans laquelle l'affection **caractérisée par** un dysfonctionnement du VEGF est un cancer, un œdème ou des vaisseaux fuyants.

**11.** Composition à utiliser selon la revendication 9, dans laquelle l'affection **caractérisée par** un dysfonctionnement du VEGF est une affection vasculoproliférative, éventuellement dans laquelle l'affection vasculoproliférative comprend une néovascularisation, une prolifération de cellules endothéliales vasculaires, une angiogenèse, une télangiectasie ou des microanévrismes.

**12.** Composition selon l'une quelconque des revendications 1 à 8, à utiliser dans la prévention ou le traitement d'un état vasculoprolifératif de l'œil, éventuellement dans lequel l'état vasculoprolifératif de l'œil est choisi parmi la dégénérescence maculaire liée à l'âge (DMLA), la rétinopathie diabétique, l'occlusion veineuse rétinienne, rétinopathie du prématuré, la télangiectasie maculaire, ou la néovascularisation choroïdienne, la vasculopathie diabétique, les maladies **caractérisées par** l'angiogenèse rétinienne et la drépanocytose.

**13.** Composition à utiliser selon l'une quelconque des revendications 9 à 12, dans laquelle la composition est formulée pour une administration topique.

**14.** Composition à utiliser selon la revendication 13, **caractérisée en ce que** la composition est formulée pour une administration topique dans l'œil, éventuellement pour une administration dans l'œil par gouttes ophtalmiques.

**15.** Kit comprenant la composition telle que définie à l'une quelconque des revendications 1 à 8.

## Figure 1

A

B

Figure 2

Figure 3

Figure 4

[A]

x4 fold enhancement

[B]

[C]

Figure 5

| R8 μg/mL | Lucentis μg/mL | R8 Molarity | Lucentis molarity | Molar ratio |
|---|---|---|---|---|
| 250 | 50 | 0.000208333 | 1.02041E-06 | 204 |
| 200 | 50 | 0.000166667 | 1.02041E-06 | 163 |
| 25 | 50 | 2.08333E-05 | 1.02041E-06 | 20 |
| 2.5 | 50 | 2.08333E-06 | 1.02041E-06 | 2 |
| 0.25 | 50 | 2.08333E-07 | 1.02041E-06 | 0.20 |
| 0.025 | 50 | 2.08333E-08 | 1.02041E-06 | 0.02 |

Figure 6

**Figure 7**

Figure 8

EP 3 672 641 B1

# Figure 9

# Figure 10

Figure 10 (continued)

## [B]

Figure 11

Figure 12

Rabbit Expt 1 & 2: Free lucentis (2h)

Figure 13

**Figure 14**

Rabbit Expt 3: Lucentis + R8 (2h)

## Figure 15

| Treatment R/L ratio | Retinal Lucentis (ng/g, ± SE) | |
|---|---|
| | 2h |
| Free lucentis control (10 mg/mL) | 18 ± 8 |
| 20/1 | 16 ± 6 |
| 200/1 | **215 ± 28** |

**Figure 16**

# Figure 17

[A]

7 days at 4°C (R8:Lucentis formulation)

Block colour: 10 mg/mL Lucentis
Checked: 1 mg/mL Lucentis
☐ 100 mg/mL Trehalose
▨ 300 mg/mL Trehalose
▨ 600 mg/mL Trehalose

7 days at 4°C (Lutrol formulation)

Block colour: 10 mg/mL Lucentis
Checked: 1 mg/mL Lucentis
☐ 100 mg/mL Trehalose
▨ 300 mg/mL Trehalose
▨ 600 mg/mL Trehalose

**Figure 17 continued**

**[B]**

**[C]**

12 hours U8:Lucentis Stability

Figure 17 continued

## [D]

Lucentis Standard

U8-Lucentis
(80:1 + Chitosan)

## Figure 18

[A]

[B]

Stability Summary

| Liquid Formulation | | | | |
|---|---|---|---|---|
| L:R Ratio | Trehalose (mg/mL) | | | |
| 0 (Lucentis only) | 100 | 200 | 300 | 600 |
| 12.5:1 | 100 | 200 | 300 | - |
| 25:1 | 100 | 200 | 300 | - |
| 50:1 | 100 | 200 | 300 | - |
| 75:1 | 100 | 200 | 300 | - |
| 100:1 | 100 | **200** | **300** | 600 |
| 140:1 | **100** | **200** | **300** | - |
| 200:1 | **100** | **200** | **300** | **600** |

| 20% Lutrol Hydrogel Formulation | | | |
|---|---|---|---|
| L:R Ratio | Trehalose (mg/mL) | | |
| 0 (Lucentis only) | 100 | 300 | 600 |
| 100:1 | 100 | 300 | 600 |
| 200:1 | 100 | 300 | 600 |

Key
- Sample stable for > 6h at 25°C
  (No aggregation observed at 600nm)

- **Sample unstable
  (Significant aggregation observed
  at 600nm)**

Figure 19

[A]

Figure 19 continued

[B]

## 12h post Hydrogel administration

## 12h post Hydrogel administration

Figure 19 continued

## [C]

Figure 20

[A]

[B]

[C]

Figure 20 continued

# [D]

Figure 21

[A]

| Treatment | Retinal Lucentis (ng/g, ± SEM) | | | |
|---|---|---|---|---|
| | 2h | 12h | 24h | 72h |
| 1. Free Lucentis (10 mg/mL) Control | 18<br>± 8 | - | - | - |
| 2. Lucentis (10 mg/mL) + R8 (200/1 ratio) | 215<br>± 28 | - | <10 | - |
| 3. Lucentis (100 mg/mL) + R8 (20/1 ratio) | 16<br>± 6 | - | - | - |
| 4. U8 gel Lucentis (10 mg/mL) + R8 (200/1 ratio) | 41823<br>± 18709 | 5282<br>± 2840 | - | - |
| 5. U8 gel Lucentis (10 mg/mL) + R8 (50/1 ratio)<br>Once Daily | 1254<br>± 460 | 23329<br>± 11096 | 19<br>± 9 | - |
| 6. Chitosan (0.1%) U8 Lucentis (10 mg/mL) + R8 (80/1) | 336<br>± 103 | 3188<br>± 1197 | 16866<br>± 1615 | 152<br>± 127 |

Figure 21 continued

[B]

0 hr

Before Fluorescein

After Fluorescein

2 hrs

Figure 22

# Figure 23

Figure 24

# Figure 25

**SPR**

Lucentis immobilised on CM5 chip surface RU=3700

Buffer: 10 mM HisCl pH 5.5, 10% Trehalose ●

Sensorgram

10 mM HEPES, pH 7.4, 150 mM NaCl, 3 mM EDTA and 0.05% v/v Surfactant P20 ●

Binding

uM R8

EP 3 672 641 B1

Figure 26

## Figure 26

[C]

0-2.5 Ratio R8: Lucentis

2.5-180 Ratio R8: Lucentis

4. ITC: No Binding

## Figure 27

# Figure 28

Investigations of Interaction between R8 and Lucentis

|   | Method | Result |
|---|---|---|
| 1 | **SEC (Size Exclusion Chromatography)** | **No binding** |
| 2 | **QCM (Quartz Crystal Microbalance)** | **No binding** |
| 3 | **SPR (Surface Plasmon resonance)** | **? Some** |
| 4 | **ITC (Isothermal titration calorimetry)** | **No binding** |
| 5 | **Dextrans** | **No binding** |
| 6 | Native/SDS PAGE | No visible bands |
| 7 | MSMF-TOF Crosslinking | too many -NH$_2$ |

## REFERENCES CITED IN THE DESCRIPTION

**Patent documents cited in the description**

- WO 1713724 A

**Non-patent literature cited in the description**

- **ROSENFELD P et al.** *NEJM,* 2006, vol. 355, 1419-1431
- **YE et al.** *Int. J. Mol. Sci.,* 2016, vol. 17 (11), 1892
- **NEMOTO et al.** *Biol Pharm Bull.,* September 2007, vol. 30 (9), 1768-72
- **MEZIERE et al.** *J. Immunol.,* 1997, vol. 159, 3230-3237
- **B. DAVIS ; E. NORMANDO ; L. GUO ; P. O'SHEA ; S. MOSS ; S. SOMAVARAPU ; M. CORDEIRO.** *Small,* 2014, vol. 10, 1575
- **F. DE COGAN ; L. J. HILL ; A. LYNCH ; P. J. MORGAN-WARREN ; J. LECHNER ; M. R. BERWICK ; A. F. A. PEACOCK ; M. CHEN ; R. A. H. SCOTT ; H. XU.** *Investig. Opthalmology Vis. Sci.,* 2017, vol. 58, 2578
- **F. OLING ; W. BERGSMA-SCHUTTER ; A. BRIS-SON.** *J. Struct. Biol.,* 2001, vol. 133, 55
- **B. CAMPOS ; S. WANG ; G. S. RETZINGER ; M. A. KAETZEL ; B. A. SEATON ; N. J. KARIN ; J. D. JOHNSON ; J. R. DEDMAN.** *Arch. Med. Res.,* vol. 30, 360
- **Y. MO ; B. CAMPOS ; T. R. MEALY ; L. COMMODORE ; J. F. HEAD ; J. R. DEDMAN ; B. A. SEATON.** *J. Biol. Chem.,* 2003, vol. 278, 2437
- **V. KHEIFETS ; R. BRIGHT ; K. INAGAKI ; D. SCHECHTMAN ; D. MOCHLY-ROSEN.** *J. Biol. Chem.,* 2006, vol. 281, 23218
- **M. VAZDAR ; E. WERNERSSON ; M. KHABIRI ; L. CWIKLIK ; P. JURKIEWICZ ; M. HOF ; E. MANN ; S. KOLUSHEVA ; R. JELINEK ; P. JUNGWIRTH.** *J. Phys. Chem. B,* 2013, vol. 117, 11530
- **K. MELIKOV ; A. HARA ; K. YAMOAH ; E. ZAITSEVA ; E. ZAITSEV ; L. V CHERNOMORDIK.** *Biochem. J,* 2015, vol. 471, 221
- **Z. WU ; Q. CUI ; A. YETHIRAJ.** *J. Phys. Chem. B,* 2013, vol. 117, 12145
- **H. KENIS ; H. VAN GENDEREN ; N. M. DECKERS ; P. A G. LUX ; L. HOFSTRA ; J. NARULA ; C. P. M. REUTELINGSPERGER.** *Exp. Cell Res.,* 2006, vol. 312, 719
- **H. KENIS ; H. VAN GENDEREN ; A. BENNAGHMOUCH ; H. A RINIA ; P. FREDERIK ; J. NARULA ; L. HOFSTRA ; C. P. M. REUTELING-SPERGER.** *J. Biol. Chem.,* 2004, vol. 279, 52623
- **J. H. TRUEBLOOD ; R. M. ROSSOMONDO ; L. A. WILSON ; W. H. CARLTON.** *Arch. Ophthalmol. (Chicago, Ill. 1960),* 1975, vol. 93, 127
- **Y. SAISHIN ; Y. SAISHIN ; K. TAKAHASHI ; R. L. E SILVA ; D. HYLTON ; J. S. RUDGE ; S. J. WIEGAND ; P. A. CAMPOCHIARO.** *J. Cell. Physiol.,* 2003, vol. 195, 241
- **L. YU ; X. WU ; Z. CHENG ; C. V. LEE ; J. LECOUTER ; C. CAMPA ; G. FUH ; H. LOWMAN ; N. FERRARA.** *IOVS,* 2008, vol. 49, 522
- **H. GERBER ; X. WU ; L. YU ; C. WIESMANN ; X. LIANG ; C. LEE ; G. FUH ; C. OLSSON ; L. DAMICO ; D. XIE.** *PNAS,* 2007, vol. 104, 3478
- **W.-C. LIANG ; X. WU ; F. V. PEALE ; C. V. LEE ; Y. G. MENG ; J. GUTIERREZ ; L. FU ; A. K. MALIK ; H.-P. GERBER ; N. FERRARA.** *J. Biol. Chem.,* 2006, vol. 281, 951
- **C. FISCHAK et al.** *J. Ophthalmology,* 2017
- **D. SCHMIDL et al.** *J. Ocular Pharmacology and Therapeutics,* 2017, vol. 33, 375-382